# EUROPÄISCHE PATENTANMELDUNG

(11) **EP 0 893 493 A2**
(43) Veröffentlichungstag der Anmeldung: **27.01.1999**
(21) Anmeldenummer: 98113137.8
(22) Anmeldetag: 15.07.1998
(51) Int. Cl.: C12N 5/10, A61K 48/00

(54) **Genetisch veränderte Zellen und deren Verwendung in der Prophylaxe oder Therapie von Erkrankungen**

(30) Priorität: 21.07.1997 DE 19731154; 26.11.1997 DE 19752299
(71) Anmelder: Hoechst Marion Roussel Deutschland GmbH, 65929 Frankfurt am Main (DE)
(72) Erfinder: Havemann, Klaus, Prof., 35041 Marburg (DE); Müller, Rolf, Prof. Dr., 35037 Marburg (DE); Sedlacek, Hans-Harald, Prof. Dr., 35041 Marburg (DE)

(57) **Zusammenfassung**

Die Erfindung bezieht sich auf Zellen zur Anwendung in der Gentherapie erhältlich durch
a) Isolierung von Zellen aus dem Blut oder zellhaltigen Flüssigkeiten des Körpers;
b) Kultivierung der in Schritt a) gewonnenen Zellen in einem Zellkulturmedium enthaltend Ganglioside, Phospholipide, Glykolipide und/oder Wachstumsfaktoren für Endothelzellen, einschließlich solcher Faktoren, welche Differenzierung, Überleben, Migration und/oder Vaskularisation beeinflussen;
c) wahlweise Immortalisierung der in Schritt a) oder b) gewonnenen Zellen durch Transformation mit einem Onkogen, Aktivierung eines Onkogens oder Inaktivierung eines Suppressorgens; und
d) wahlweise Transfektion der in Schritt a) und b) oder in Schritt c) gewonnenen Zellen mit einem Nukleinsäurekonstrukt für die Gentherapie, enthaltend ein Effektorgen, welches durch geeignete Promotorsysteme zielzellspezifisch, zellzyklusspezifisch, virusspezifisch und/oder durch Hypoxie aktiviert werden kann;
und die Verwendung dieser Zellen zur Herstellung eines Heilmittels zur Behandlung einer Erkrankung ausgewählt aus der Gruppe enthaltend Tumore, Leukämie, Autoimmunerkrankungen, Allergien, Arthritiden, Entzündungen, Organabstoßungen, Transplantat gegen Wirt-Reaktionen, Blutgerinnungserkrankungen, Kreislauferkrankungen, Blutarmut, Infektionen, Hormonerkrankungen und ZNS-Schäden.

## Beschreibung

Die vorliegende Erfindung bezieht sich auf Zellen zur Anwendung in der Gentherapie erhältlich durch
a) Isolierung von mononukleären Zellen aus dem Blut oder zellhaltigen Flüssigkeiten des Körpers;
b) Kultivierung der in Schritt a) gewonnenen Zellen in einem Zellkulturmedium enthaltend Ganglioside, Phospholipide, Glykolipide und/oder Wachstumsfaktoren für Endothelzellen, einschließlich solcher Faktoren, welche Differenzierung, Überleben, Migration und/oder Vaskularisation beeinflussen;
c) wahlweise Immortalisierung der in Schritt a) oder b) gewonnenen Zellen durch Transformation mit einem Onkogen, Aktivierung eines Onkogens oder Inaktivierung eines Suppressorgenes; und
d) wahlweise Transfektion der in Schritt a) und b) oder in Schritt c) gewonnenen Zellen mit einem Nukleinsäurekonstrukt für Gentherapie, enthaltend ein Effektorgen, welches durch geeignete Promotorsysteme zielzellspezifisch, zellzyklusspezifisch, virusspezifisch und/oder durch Hypoxie aktiviert werden kann.

### 1) Einleitung

Die Verabreichung von in vitro transfizierten somatischen Zellen, transduziert zur Expression eines Wirkstoffes, ist eine derzeit breit präklinisch wie auch klinisch in Prüfung befindliche Methode der Gentherapie. Unterschiedliche Zellen werden hierbei verwendet, einschließlich Fibroblasten, Lymphozyten, Keratinozyten und Tumorzellen.

Endothelzellen kamen erstmals 1989 für diesen Zweck zur Anwendung. Hierzu wurden die Endothelzellen mit Hilfe eines retroviralen Vektors (Zwiebel et al., Science 243: 220 (1989)) in vitro transfiziert zur Expression eines Wirkstoffes.

Derartig transduzierte Endothelzellen, in vitro angewachsen an Blutgefäßprothesen aus Kunststoff, waren nach in vivo Transplantation dieser Prothesen in der Lage, das Transgen zu exprimieren (Zwiebel et al., Science 243: 220 (1989), Wilson et al. Science 244: 1344 (1989)). In Konsequenz wurde von Zwiebel und Wilson vorgeschlagen, transduzierte Endothelzellen, adhärierend an einen Kunststoff oder Kollagenträger, zum Zwecke einer Gentherapie Patienten zu verabreichen. Experimentell durchgeführt wurde dieser Vorschlag von Nathan et al. (PNAS USA 92: 8130 (1995)).

In Erweiterung dieses Vorschlages wurde von Nabel et al. (Science 244: 1342 (1989)) erstmals die Möglichkeit der Verabreichung einer Zellsuspension von transduzierten Endothelzellen in den Blutstrom hinein als Weg der Gentherapie dargestellt. Die Autoren konnten zeigen, daß Endothelzellen, welche aus Gefäßen eines lebenden Saugers durch Abschaben gewonnen und in vitro zur Expression eines Reportergenes transduziert worden waren, nach lokaler Verabreichung z. B. in Blutgefäße mit einem Endothelzellschaden dort anwachsen und das Reportergen exprimieren. Aufgrund dieser Ergebnisse beschrieben die Autoren die Möglichkeit, genetisch modifizierte Endothelzellen zum Zwecke der Gentherapie zu verabreichen, wobei Wirkstoffe von diesen Endothelzellen direkt in die Blutzirkulation zum Zwecke der Therapie von systemischen oder von Erbleiden abgegeben werden. Diese Idee wurde von Bernstein et al. (FASEB J. 4: 2665 (1990)) weiterentwickelt. Lungenendothelzellen wurden in vitro zur Expression eines Wirkstoffes mit Plasmiden transfiziert und Nacktmäusen nachfolgend intraperitoneal, intravenös, subkutan oder unter die Nierenkapsel injiziert. In den derartig behandelten Tieren wurde der von den Endothelzelltransplantaten produzierte Wirkstoff lokal (in Zysten der Niere) oder im Blut (nach i.p., i.v. oder s.c. Applikation) nachgewiesen, was die Brauchbarkeit der in vivo Verabreichung von in vitro transduzierten Endothelzellen für die Gentherapie bewies.

Nachfolgend zeigten Zwiebel et al., 1992 (Internationale Patentanmeldung mit der Veröffentlichungsnummer WO93/13807) und Ojeifo et al. (Cancer Res. 55: 2240 (1995)) an mehreren Beispielen die Einsatzmöglichkeit der Methode der Verabreichung von in vitro transduzierten Endothelzellen in den Blutkreislauf zum Zwecke der Gentherapie.

Zwiebel et al. (1992) transduzierten in vitro humane Nabelschnurendothelzellen und Endothelzellen aus dem Fettgewebe von Ratten mit einem retroviralen Vektor zur Expression von einem Wirkstoff und injizierten diese Endothelzellen intravenös in Tiere, in denen durch die Injektion von bestrahlten FGF-sekretierenden Zellen vorab ein lokaler Gefäßschaden und Angiogenese erzeugt worden waren. Sie konnten zeigen, daß die injizierten Endothelzellen am Ort des Gefäßschadens und der Angiogenese lokalisieren und dort den Wirkstoff exprimieren. Vor diesem Hintergrund beanspruchten die Autoren in ihrer Patentanmeldung die Verwendung von in vitro transduzierten Endothelzellen für die Expression von Adenosindeaminase, Blutgerinnungsfaktoren, hematopoietische Wachstumsfaktoren, Zytokine, Antithrombotika, Enzyminhibitoren und Hormone.

Parallel zu diesen Arbeiten wurde von anderen Autoren sowohl die Technik zur Isolierung von Endothelzellen verbessert als auch das Wanderungsverhalten von in vitro transduzieren Endothelzellen näher studiert.

So konnten Messina et al. (PNAS USA 89: 12018 (1992)) zeigen, daß sich in vitro transfizierte Endothelzellen nach Injektion in den Kreislauf sowohl an die intakte Endothelzellschicht anheften als auch in diese hinein integrieren können. Eine ausschließliche Lokalisation von intravaskulär verabreichten Endothelzellen in Zonen mit Gefäßschaden und Angiogenese konnte mit diesen Ergebnissen also ausgeschlossen werden. Andererseits konnte gezeigt werden, daß sich im Gemisch mit Tumorzellen injizierte, in vitro transfizierte Endothelzellen an der Angiogenese des Tumorgefäßbettes beteiligen (Lal et al., PNAS USA 91: 9695 (1994), Nam et al. Brain Res. 731: 161 (1996)). Hierdurch bedingt weisen Tumorzellen, transplantiert im Gemisch mit Endothelzellen, in vivo einen deutlichen Wachstumsvorteil auf (Stopeck et al., Proc. Am. Assoc. Cancer Res. 38: 265 (1997)).

Andererseits können transduzierte Endothelzellen lokal z. B. in das Hirn oder in einen Hirntumor verabreicht, durch Expression des vom Transgen kodierten Wirkstoffes pharmakologisch aktiv bzw. antitumoral wirksam sein (Nam et al. Brain Res. 731: 161 (1996), Quinonero et al., Gene Ther. 4: 111 (1997)). Beispielsweise applizierten Robertson et al. (Proc. Am. Assoc. Cancer Res. 38: 382 (1997)) humane Endothelzellen (HUVEC), in vitro transduziert mit einem AV-Vektor zur Expression von HSV-TK, im Gemisch mit humanen Ovarialkarzinomzellen. Nach Gabe von Ganciclovir, das im Tumor durch die HSV-TK zu einem Zytostatikum aktiviert wird, konnten sie eine deutliche Tumorregression bei Nacktmäusen beobachten.

Die Verwendung von Endothelzellen als zellulären Träger von Transgenen in der Gentherapie ist bislang jedoch durch zwei wesentliche Problembereiche erheblich eingeschränkt gewesen:
- Die Gewinnung von geeigneten Endothelzellen in ausreichender Anzahl hat sich bislang als äußerst schwierig erwiesen.
   Allogene Endothelzellen sind zwar relativ einfach aus der Nabelschnur oder aus Zellkulturen zu gewinnen, durch ihre Immunogenität im Empfänger sind sie jedoch nur eingeschränkt verwendbar, zum anderen ist ihre Vermehrung in der Zellkultur nur in beschränktem Maße möglich.
   Autologe Endothelzellen können zwar beispielsweise mechanisch durch das "Ausschaben" von Krampfadern oder aus Fettgewebe gewonnen werden. Diese Gewinnungsart ist jedoch nicht bei allen Patienten möglich und bedeutet zudem eine erhebliche Beeinträchtigung des Patienten. Daher wurden alternativ Angioblasten oder Vorläuferzellen von Endothelzellen aus dem peripheren Blut gewonnen (Asahara et al., Science 275: 964 (1997)). Die hierzu notwendige Blutentnahme ist für Patienten zwar wenig belastend, die Isolierung der vermeintlichen Angioblasten aus mononukleären Blutzellen und die Differenzierung dieser Angioblasten zu Endothelzellen ist jedoch sehr aufwendig. So werden aus Blutleukozyten (isoliert mit Hilfe der Dichte-Gradientenzentrifugation) CD34⁺ oder Flk1⁺ mononukleäre Blutzellen, die im Blut in nur geringer Konzentration (≤ 0,1 %) vorhanden sind, durch Immunadsorption an trägergebundene monoklonale Antikörper (spezifisch für CD34 oder Flk-1) angereichert. Nachfolgend werden diese Zellen in Gewebeschalen beschichtet mit Kollagentyp 1 oder Fibronektin für etwa 4 Wochen im Rinderhirn-haltigem Kulturmedium zur Differenzierung in Endothelzellen wie auch zur Vermehrung inkubiert. Die Vermehrung dieser Zellen ist jedoch nur sehr beschränkt möglich. Des weiteren wirft die Inkubation der Endothelzellen mit Hirnsubstanz, z. B. mit Rinderhirn, erhebliche Sicherheitsprobleme auf.
- Die Wanderung der Endothelzellen und die selektive Expression des Transgenes im gewünschten Zielgebiet ist nicht ausreichend kontrollierbar.
   Nach intravaskularer Verabreichung der Endothelzellen lokalisieren diese (wie bereits oben beschrieben) sowohl in Regionen der Angiogenese als auch an und in die ruhende Endothelzellschicht. Des weiteren ist unklar, ob Endothelzellen, die in der Zellkultur aus Vorläuferzellen entstanden sind, sich in vivo nach Injektion wieder in Vorläuferzellen rückdifferenzieren und über den gesamten Organismus verteilen können.

Mit der vorliegenden Erfindung werden nun beide wesentlichen Problembereiche gelöst.

### 2) Allgemeine Beschreibung der Erfindung

A) Die Erfindung besteht in
   1) einer verbesserten, d. h. einfachen und sicheren Methode zur Isolierung und Kultivierung von mononukleären Zellen, insbesondere von Endothel-Vorläuferzellen aus dem Blut und anderen zellhaltigen Flüssigkeiten des Körpers und der Verwendung dieser Zellen für die Prophyllaxe oder Therapie einer Erkrankung.
   2) wahlweise in einer zell-, insbesondere endothelzellspezifischen, ggf. pharmakologisch kontrollierbaren Transformation dieser Zellen, so daß mit Hilfe der Zellkultur leicht größere Mengen von solchen Zellen gewonnen werden können
   3) der Herstellung von Zellen, insbesondere Endothelzellen, als Vektoren für Effektorgene dergestalt, daß in diese Zellen hergestellt nach 1) oder 1) und 2) mindestens ein Effektorgen eingefügt ist, das durch die Wahl geeigneter Promotorsysteme zell-, insbesondere endothelzellspezifisch und ggf. durch Hypoxie, zellzyklusspezifisch und/oder virusspezifisch exprimiert wird
   4) der Verabreichung dieser so gewonnenen genetisch veränderten Zellen, insbesondere Endothelzellen zur Prophylaxe oder Therapie einer Erkrankung.
B) Ein Gegenstand der Erfindung sind daher Zellen zur Anwendung in der Gentheraphie erhältlich durch
   a) Isolierung von mononukleären Zellen aus dem Blut oder zellhaltigen Flüssigkeiten des Körpers;
   b) Kultivierung der in Schritt a) gewonnenen Zellen in einem Zellkulturmedium enthaltend Ganglioside, Phospholipide, Glykolipide und/oder Wachstumsfaktoren Wachstumsfaktoren für Endothelzellen, einschließlich solcher Faktoren, welche Differenzierung, Überleben, Migration und/oder Vaskularisation beeinflussen;
   c) wahlweise Immortalisierung der in Schritt a) oder b) gewonnenen Zellen durch Transformation mit einem Onkogen, Aktivierung eines Onkogens oder Inaktivierung eines Suppressorgenes;
   d) Transfektion der in Schritt a) und b) oder in Schritt c) gewonnenen Zellen mit einem Nukleinsäurekonstrukt für die Gentherapie, enthaltend ein Effektorgen, welches durch geeignete Promotorsysteme zielzellspezifisch, zellzyklusspezifisch, virusspezifisch und/oder durch Hypoxie aktiviert werden kann.

Weitere Gegenstände der Erfindung, Ausführungsformen der Erfindung sowie entsprechende Beispiele werden im folgenden beschrieben.

### 3) Herstellung von Endothelzellen

### 3.1.) Isolierung und Kultivierung von Vorläuferzellen von Endothelzellen

Das erfindungsgemäße Verfahren der Isolierung von Vorläuferzellen von Endothelzellen ist in folgende Abschnitte zu zergliedern:

Zellhaltige Körperflüssigkeiten werden mit den dem Fachmann bekannten invasiven Verfahren aus den jeweiligen Organen entnommen. Zu diesen zellhaltigen Körperflüssigkeiten gehören beispielsweise
- Blut gewonnen aus Venen, Kapillaren, Arterien oder der Nabelschnur bzw. Plazenta
- Knochenmarkzellsuspensionen
- Milzzellsuspensionen
- Lymphknotenzellsuspensionen
- Peritonealzellsuspensionen
- Pleuralzellsuspensionen
- Lymphe
- Bindegewebsflüssigkeit (austretend z. B. auf die Oberfläche einer oberflächlich z. B. mechanisch geschädigten Epidermis).

Erythrozyten, Granulozyten und andere Zellkomponenten werden aus diesen Körperflüssigkeiten durch Dichte-Gradientenzentrifugation und Thrombozyten durch Differentialzentrifugation entsprechend den dem Fachmann bekannten Methoden abgetrennt.

Die so isolierten mononukleären (kernhaltigen) Zellen werden in Serum enthaltendem Zellkulturmedium suspendiert. Das verwandte Zellkulturmedium enthält die weiter unten aufgeführten Ganglioside,Phospholipide und/oder Wachstumsfaktoren.

In einer besonderen Ausführungsform dieser Erfindung werden die isolierten, mononukleären (kernhaltigen) Zellen in diesem Zellkulturmedium kultiviert und zu endothelzellähnlichen Zellen differenziert.

In einer weiteren besonderen Ausführungsform dieser Erfindung werden die isolierten kernhaltigen mononukleären Zellen mit einem Antikörper gegen Monozyten/Makrophagen typische Oberflächenmarker (CD11, CD11b, CD13, CD14, CD34, CD64, CD68) der an Polysaccharid beschichtete Eisen- bzw. Eisenoxyd-Partikel gekoppelt ist, inkubiert, gewaschen, und anschließend die so beladenen Zellen mit Hilfe eines Magneten gewonnen. Die Zellen werden in ein Zellkulturmedium gegeben, welches die weiter unten aufgeführten Ganglioside, Phosholipide und/oder Wachstumsfaktoren enthält und in vitro weiter vermehrt und zu Endothelzellen differenziert. Die Immortalisierung und/oder Transfektion wird nach Vermehrung und/oder Differenzierung der Zellen in vitro durchgeführt.

In einer weiteren Ausführungsform dieser Erfindung werden die isolierten kernhaltigen Zellen in dem genannten Zellkulturmedium für > 1 Stunde zur weiteren Differenzierung und Vermehrung vorinkubiert. Unter diesen Bedingungen entwickeln die als Endothel-Vorläuferzellen erkannten Zellen zunehmend für Monozyten/Makrophagen typische Oberflächen-Marker (CD11, CD11b, CD13, CD14, CD34, CD64, CD68). Diese Zellen werden anschließend isoliert, beispielsweise werden sie mit einem Antikörper der gegen diese Monozytenmarker gerichtet ist (z.B. CD11, CD14) und der an dextranbeschichtete Eisenpartikel gekoppelt ist, mit Hilfe eines Magneten gewonnen. Die Zellen werden in vitro weiter vermehrt und zu Endothelzellen differenziert.

Alternativ hierzu werden aus nichtadhärierenden mononukleären Zellen wie beispielsweise von Asahara et al., Science 275, 964 (1997) beschrieben, die CD34-positiven Zellen (hämatopoetische Stammzellen) isoliert und in vitro weiter vermehrt und zu Endothelzellen differenziert.

In einer besonderen Ausführungsform der Erfindung werden die isolierten kernhaltigen Zellen in Zellkulturmedium suspendiert und die verbleibenden phagozytierenden Zellen (z.B. Monozyten, Makrophagen, Granulozyten) durch Adhärieren an der Oberfläche bzw. durch Phagozytose von proteinbeladenen, dextran-beschichteten Eisenpartikeln mit Hilfe eines Magneten und/oder durch Gegenstromzentrifugation entsprechend den dem Fachmann bekannten Verfahren entfernt und die verbleibenden CD34-positiven Zellen enthaltenden mononukleären Zellen in dem erfindungsgemäßen Zellkulturmedium kultiviert und zu endothelzellähnlichen Zellen differenziert.

Die Zellkulturgefäße können mit einer Extrazellular-Matrixkomponente (siehe Attachment and Matrix Factors, z.B. von Fa. Sigma) wie beispielsweise Fibronektin, beschichtet sein. Dem Zellkulturmedium werden entweder Ganglioside, Phospholipide und/oder Glykolipide zugegeben und/oder aber bevorzugt entsprechend dieser Erfindung Wachstumsfaktoren für Endothelzellen, beispielsweise
- Vascular endothelial Growth Factor (VEGF) und/oder andere KDR oder FLt-Liganden wie
- Fibroblast Growth Factor (FGFα, FGFβ) und/oder
- Epidermal Growth Factor (EGF) und/oder
- Insulin-like Growth Factor (IGF-1, IGF-2) und/oder
- β-Endothelial Cell Growth Factor (ECGF) und/oder
- Endothelial Cell Attachment Factor (ECAF) und/oder
- Interleukin-3 (IL-3) und/oder
- GM-CSF und/oder
- G-CSF und/oder
- Interleukin-4 (II-4) und/oder
- Interleukin-1 (II-1) und/oder
- Colony stimulating factor (CSF-1) und oder
- Interleukin-8 (II-8) und/oder
- Platelet derived growth factor (PDGF) und/oder
- IFNγ und/oder
- Oncostatin M und/oder
- LIF und/oder
- B61 und/oder
- Platelet derived endothelial cell growth factor (PDEGF) und/oder
- Stem Cell Factor (SGF) und/oder
- Transforming Growth Factor β (TGF-β) und/oder
- Angiogenin und/oder
- Pleiotrophin und/oder
- Flt-3-Ligand (FL) und/oder
- -Fie-2-Liganden wie z.B. Angiopoietin-1 und/oder
   Stromal derived Factor-1 (SDF-1) und/oder
   Midkine
zugesetzt.

Die nach einer Zeit, ausgewählt zwischen 6 Stunden und 8 Wochen, herangewachsenen Zellen werden erfindungsgemäß weiterbehandelt.

Die so isolierten Endothelzellen können auch direkt zur Förderung der Endothelisierung verletzter Gefäße und zur Förderung der Angiogenese eingesetzt werden.

### 3.2.) Isolierung von Endothelzellen

Alternativ zu der erfindungsgemäßen Methode, dargestellt in Abschnitt 3.1.), können Endothelzellen jedoch auch mit den dem Fachmann bekannten Methoden zum Beispiel aus Fettgewebe, durch Ausschabung von Venen oder durch Ablösung vom Nabelschnurendothel gewonnen werden. Ihre Kultivierung erfolgt wie bereits im Abschnitt 3.1.) beschrieben.

### 4) Immortalisierung von Endothelzellen

Entsprechend dieser Erfindung kann in eine oder mehrere erfindungsgemäße nichtadhärente mononukleäre Zelle oder Endothelzelle eine Nukleotidsequenz (Komponente a) für ein Protein eingeführt werden, welches bewirkt, daß diese Zellen fortlaufend den Zellteilungszyklus durchlaufen und damit zu einer nicht alternden, "permanent" sich teilenden Zellinie werden. Solche immortalisierenden Nukleotidsequenzen bzw. Gene sind bereits bekannt. Zu diesen Nukleotidsequenzen gehören beispielsweise Onkogene. Entsprechend dieser Erfindung können die Onkogene zellulären oder viralen Ursprungs sein. Beispiele für zelluläre Onkogene wurden bereits von Wynford-Thomas, J. Pathol. 165: 187 (1991); Harrington et al., Curr. Opin. Genet. Developm. 4: 120 (1994); Gonos et al., Anticancer Res. 13: 1117 (1993) und Baserga et al., Cancer Surveys 16: 201 (1993) übersichtlich dargestellt.
Derartige Onkogene können in die Zelle mit dem Fachmann bekannten Methoden eingeführt werden. Zellen tragen in ihrem Genom jedoch auch Protoonkogene, welche entsprechend dieser Erfindung in der Zelle mit dem Fachmann bekannten Methoden aktiviert, d. h. in Onkogene verwandelt werden können.

In einer besonderen Ausführungsform dieser Erfindung stellt die Komponente a) eine Nukleotidsequenz dar, welche ein Protein kodiert, das das Protein eines Suppressorgenes inaktiviert.

Beispiele für Suppressorgene sind bereits übersichtlich dargestellt worden von Karp und Broder, Nature Med. 4: 309 (1995); Skuse und Ludlow, The Lancet 345: 902 (1995); Duan et al., Science 269: 1402 (1995); Hugh et al., Cancer Res. 55: 2225 (1995); Knudson, PNAS USA 90: 10914 (1993)).

Beispiele für ein Gen (Komponente a), das für ein Protein kodiert, welches das Expressionsprodukt eines Suppressorgenes inaktiviert:

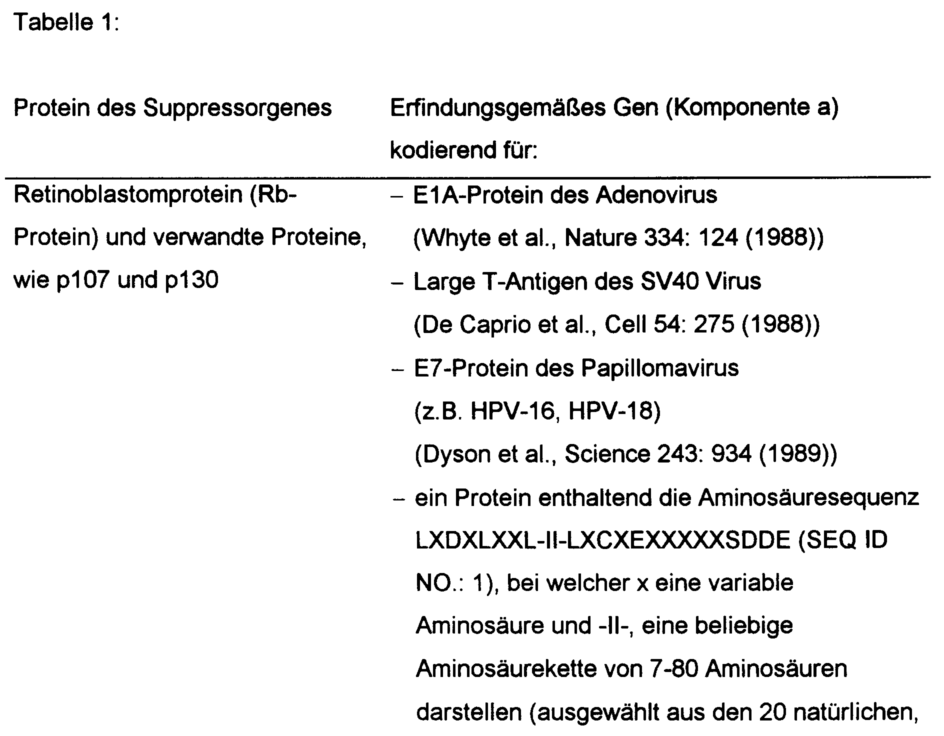

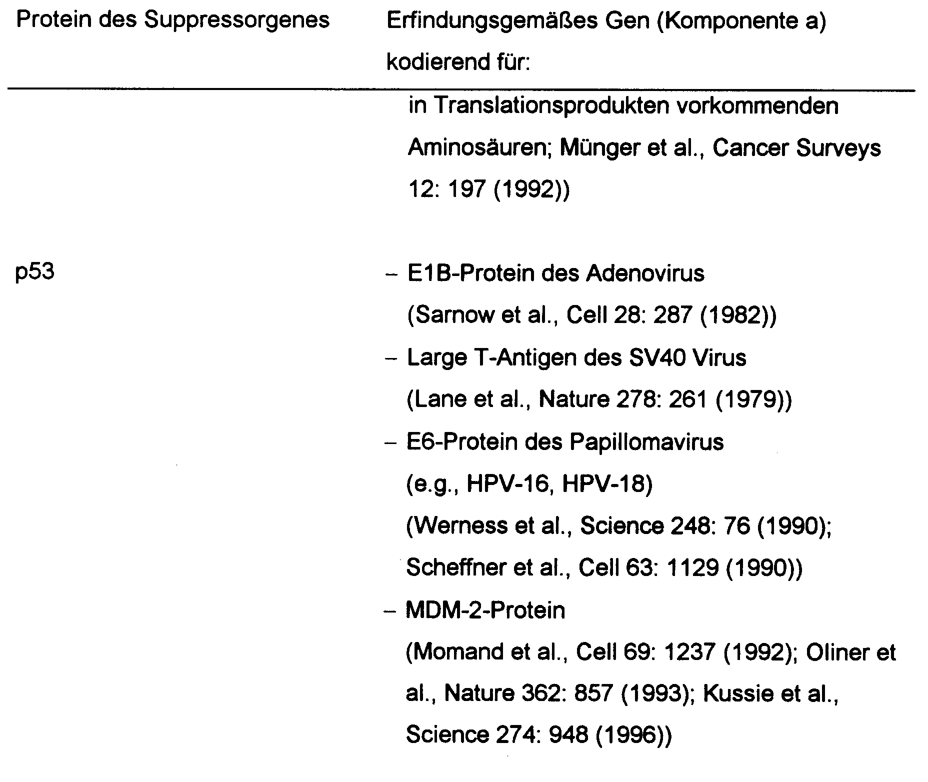

In einer weiteren besonderen Ausführungsform dieser Erfindung stellt die Komponente a) eine mutierte Nukleotidsequenz für ein Protein der Zellzyklusregulation dar, das durch die Mutation so verändert ist, daß es zwar noch voll den Zellzyklus aktivieren kann, aber in dieser Funktion nicht mehr durch zelluläre Inhibitoren inhibierbar ist. Im Sinne dieser Erfindung gehören hierzu mutierte Nukleotidsequenzen kodierend für Cyclin-abhängige Kinasen, welche trotz der Mutation ihre Kinaseaktivität beibehalten, aber die Bindefähigkeit an die zellulären cdk-Inhibitoren verloren haben.

Beispiele für die Komponente a) sind:
- cdk-4 derartig mutiert, daß p16, p15 und/oder p21 nicht mehr inhibieren können
- cdk-6 derartig mutiert, daß p15 und/oder p18 nicht mehr inhibieren können
- cdk-2 derartig mutiert, daß p21 und/oder p27 und/oder WAF-1 nicht mehr inhibieren können.

Beispielsweise kann die Mutation des cdk4 den Austausch eines Arginins durch ein Cystein in der Position 24 darstellen, so daß dieses mutierte cdk4 Kinaseaktivität aufweist, aber durch p15 und p16 nicht mehr inhibierbar ist (Wölfel et al., Science 269: 1281 (1995)).

In einer weiteren Ausführungsform dieser Erfindung stellt die Komponente a) ein transformierendes Gen dar, dessen Expression von einem selbstverstärkenden Promotorelement, ggf. in Kombination mit einem pharmakologisch kontrollierbaren Promotor (siehe hierzu Abschnitt 6.4), reguliert wird.

In einer weiteren besonderen Ausführungsform dieser Erfindung wird in die Endothelzellen, besonders aber auch in die Vorläuferzellen von Endothelzellen oder in Zellen eines Zellgemisches, das einen Anteil an Endothelzellen oder Vorläuferzellen von Endothelzellen oder einen, im Vergleich zum Blut erhöhten Anteil von CD34, CD11, CD11b, CD14, CD13, CD64 und CD68 positiven Zellen enthält, eine Nukleotidsequenz eingefügt, welche besteht aus einer endothelzellspezifischen Promotor- oder Enhancersequenz (Komponente b) und der Komponente a), wobei die Transkription der Komponente a) durch Bindung von Transkriptionsfaktoren der Endothelzelle an die Komponente b) aktiviert wird.

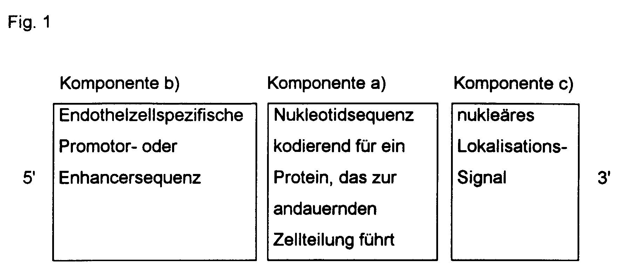

Zum besseren Verbleib des Expressionsproduktes der Komponente a) im Zellkern kann an die Komponente a) ein nukleäres Lokalisations-Signal angefügt werden (Komponente c). Die hieraus resultierende Anordnung der Komponenten ist beispielhaft in Fig. 1 dargestellt.

Durch Einführung eines Nukleinsäurekonstruktes, enthaltend die in Fig. 1 dargestellten Komponenten, werden nur Endothelzellen und Vorläufer von Endothelzellen enthalten in einem heterogenen Zellgemisch in ein immortalisiertes Stadium, d. h. in sich permanent teilende Zellen, überführt, so daß nach wenigen Tagen diese Endothelzellen anteilsmäßig in der Zellkultur dominieren und nach einer von den Kultivierungsbedingungen abhängigen, jedoch überschaubaren Zeit ausschließlich in der Zellkultur vorhanden sind.

Mit diesen erfindungsgemäßen Nukleinsäurekonstrukten und Verfahren können somit in einer relativ kurzen Zeit mit geringem Aufwand auch aus wenigen Endothelzellen oder aus wenigen Vorläuferzellen von Endothelzellen auch dann, wenn sie in einem heterogenen Zellgemisch vorliegen, große Mengen einheitlicher Endothelzellen für die Prophylaxe oder Therapie hergestellt werden.

### 5) Herstellung von genmodifizierten Endothelzellen für die Prophylaxe und/oder Therapie

Entsprechend der Erfindung ist in die nach einem der erfindungsgemäßen Verfahren gewonnenen Endothelzellen ein Nukleinsäurekonstrukt einzuführen, welches mindestens folgende Komponenten enthält:
- einen Promotor (Komponente d)
- ein Strukturgen kodierend für einen Wirkstoff oder ein Enzym (Komponente e)

### 6) Auswahl der Promotorsequenzen

Im Sinne der Erfindung sind als Promotorsequenzen Nukleotidsequenzen zu verwenden, welche nach Bindung von Transkriptionsfaktoren die Transkription eines am 3'-Ende benachbart gelegenen Transgenes, wie beispielsweise eines Strukturgenes, aktivieren. Im Sinne der Erfindung wird in die Endothelzelle mindestens eine endothelzellspezifische Promotorsequenz (Komponente b) und/oder Komponente d) eingefügt. Diese endothelzellspezifische Promotorsequenz kann mit mindestens einer weiteren Promotorsequenz kombiniert werden. Die Wahl der mit dem endothelzellspezifischen Promotor zu kombinierenden Promotorsequenz(en) richtet sich nach der zu behandelnden Erkrankung. So kann die zusätzliche Promotorsequenz uneingeschränkt, endothelzellspezifisch, unter bestimmten metabolischen Bedingungen, wie beispielsweise durch Hypoxie induzierbar oder durch ein Pharmakon induzierbar oder abschaltbar, virusspezifisch und/oder zellzyklusspezifisch aktivierbar sein. Derartige Promotoren wurden bereits in den Patentanmeldungen EP95931204.2; EP95930524.4; EP95931205.9; EP95931933.6; EP96110962.2; DE19704301.1, EP97101507.8; EP97102547.3; DE19710643.9 und EP97110995.8 aufgeführt. Auf diese Patentanmeldungen wird Bezug genommen. Zu den auszuwählenden Promotorsequenzen gehören beispielsweise

### 6.1.) Uneingeschränkt aktivierbare Promotoren und Aktivatorsequenzen,

wie beispielsweise
- der Promotor der RNA-Polymerase III
- der Promotor der RNA-Polymerase II
- der CMV-Promotor und -Enhancer
- der SV40 Promotor

### 6.2.) Metabolisch aktivierbare Promotor- und Enhancersequenzen,

wie beispielsweise der durch Hypoxie induzierbare Enhancer (Semenza et al., PNAS 88: 5680 (1991), McBurney et al., Nucl. Acids Res. 19: 5755 (1991)).

### 6.3.) Zellzyklusspezifisch aktivierbare Promotoren.

Solche sind beispielsweise der Promotor des cdc25B Gens, des cdc25C Gens, des Cyclin A Gens, des cdc2 Gens, des B-myb Gens, des DHFR-Gens, des E2F-1 Gens oder aber Bindesequenzen für während der Zellproliferation auftretende oder aktivierte Transkriptionsfaktoren. Zu diesen Bindesequenzen gehören beispielsweise Bindesequensen für c-myc-Proteine. Zu diesen Bindesequenzen sind Monomere oder Multimere der als Myc E-Box bezeichneten Nukleotidsequenz (5'-GGAAGCAGACCACGTGGTCTGCTTCC-3' (SEQ ID NO.: 2); Blackwood and Eisenmann, Science 251: 1211 (1991)) zu zählen.

### 6.4.) Selbstverstärkende und/oder pharmakologisch kontrollierbare Promotoren.

Im einfachsten Falle kann bei der Kombination von gleichen oder unterschiedlichen Promotoren ein Promotor induzierbar sein, beispielsweise in Form eines durch Tetrazyklin aktivierbaren bzw. abschaltbaren Promotors in Form des Tetracyclin-Operators in Kombination mit einem entsprechenden Repressor.

Entsprechend der Erfindung kann der Promotor jedoch auch selbstverstärkend sein mit oder auch ohne einer pharmakologisch kontrollierbaren Promotoreinheit.

Derartige selbstverstärkende un/oder pharmakologisch kontrollierbare Promotoren wurden bereits in der Patentanmeldung DE19651443.6 beschrieben, auf welche ausdrücklich Bezug genommen wird.

### 6.5.) Endothelzellspezifsch aktivierbare Promotoren

Hierzu zählen Promotoren oder Aktivatorsequenzen aus Promotoren oder Enhancern von solchen Genen, die für Proteine bevorzugt gebildet in Endothelzellen kodieren.

Im Sinne der Erfindung sind Promotoren der Gene für folgende Proteine beispielsweise zu verwenden:
- Hirnspezifischer, endothelialer Glucose-1-Transporter
- Endoglin
- VEGF-Rezeptor-1 (flt-1)
- VEGF-Rezeptor-2 (flk-1, KDR)
- tie-1 oder tie-2
- B61-Rezeptor (Eck-Rezeptor)
- B61
- Endothelin, im speziellen Endothelin B oder Endothelin-1
- Endothelin-Rezeptoren, insbesondere der Endothelin B-Rezeptor
- Mannose-6-Phosphat-Rezeptoren
- von Willebrand Faktor
- IL-1α, IL-1β
- IL-1-Rezeptor
- Vascular Cell Adhesion Molecule (VCAM-1)
- Interstitial Cell Adhesion Molecule (ICAM-3)
- synthetische Aktivatorsequenzen
Als Alternative zu natürlichen endothelzellspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist (Lee et al., Biol. Chem. 16188 (1991), Dormann et al., J. Biol. Chem. 1279 (1992) und Wilson et al., Mol. Cell Biol. 4854 (1990).

### 7) Kombination von gleichen oder unterschiedlichen Promotoren

Die Kombination von gleichen Promotoren erfolgt beispielsweise durch hintereinander Verknüpfung von mehreren Promotoren in der Leserichtung von 5' nach 3' der Nukleotidsequenz.

Zur Kombination von gleichen oder unterschiedlichen Promotoren werden bevorzugterweise jedoch Technologien eingesetzt, welche bereits in den Patentanmeldungen GB9417366.3; EP97101507.8; EP97102547.3; DE19710643.9; DE19617851.7; DE19639103.2 und DE19651443.6 im Detail beschrieben wurden. Auf diese Patentanmeldungen wird im Rahmen dieser Erfindung ausdrücklich Bezug genommen. Beispiele für derartige Technologien sind

### 7.1.) Chimäre Promotoren

Ein chimärer Promotor stellt die Kombination einer stromaufwärtsgelegenen zellspezifisch, metabolisch oder virusspezifisch aktivierbaren Aktivatorsequenz mit einem stromabwärts gelegenen Promotormodul dar, welches die Nukleotidsequenz CDE-CHR oder E2FBS-CHR enthält, an welche suppressive Proteine binden, die hierdurch die Aktivierung der stromaufwärts gelegenen Aktivatorsequenz in G₀- und G₁-Phase des Zellzyklus hemmen können (GB9417366.3; Lucibello et al., EMBO J., 12 (1994)).

Weiterführende Untersuchungen zur Funktionsweise, insbesondere des Promotorelementes CDE-CHR, ergaben, daß die durch das CDE-CHR Element zellzyklusabhängige Regulation einer stromaufwärts gelegenen Aktivatorsequenz weitgehend davon abhängig ist, daß die Aktivierungssequenz von Transkriptionsfaktoren mit glutaminreichen Aktivierungsdomänen aktiviert wird (Zwicker et al., Nucl. Acids Res., 3822 (1995)).

Zu derartigen Transkriptionsfaktoren gehört beispielsweise Sp1 und NF-Y.

Dieses schränkt konsequenterweise die Verwendung des Promotorelementes CDE-CHR für chimäre Promotoren ein. Gleiches ist für das Promotorelement E2F-BS-CHB des B-myb Genes anzunehmen (Zwicker et al., Nucl. Acids Res. 23, 3822 (1995)).

### 7.2.) Hybride Promotoren

Die hybriden Promotoren sind in der Patentanmeldung DE19639103.2 bereits beschrieben worden. Für die Kombination des endothelzellspezifischen Promotors mit mindestens einem weiteren Promotor wird beispielsweise ein Genkonstrukt gewählt, welches insgesamt die folgenden Komponenten enthält:

Die Nukleotidsequenz des endothelzellspezifischen Promotors in einer Form, bei welcher mindestens eine Bindestelle für einen Transkriptionsfaktor mutiert ist. Durch diese Mutation wird die Initiation der Transkription des Effektorgenes blockiert.

Ein Transgen, das als Effektorgen für einen Wirkstoff kodiert.

Mindestens eine weitere unspezifisch, zellspezifisch, virusspezifisch, durch Tetrazyklin und/oder zellzyklusspezifisch aktivierbare Promotor- oder Enhancersequenz, welche die Transkription mindestens eines Genes für mindestens einen Transkriptionsfaktor aktiviert, welcher derartig mutiert ist, daß er an die mutierte(n) Bindestelle(n) in dem endothelzellspezifischen Promotor binden und diesen aktiveren kann.

In einer beispielhaften Ausführungsform dieser Erfindung kann die Mutation in der Promotorsequenz z. B. eine Mutation der TATA-Box des cdc25B Promotors darstellen.

Die Mutation der TATA kann beispielsweise TGTATAA sein. Durch diese Mutation wird die DNA-Bindungsstelle des normalen TATA-Box bindenden Proteins (TBP) nicht mehr erkannt und das Effektorgen kann nicht mehr effizient transkribiert werden. Entsprechend muß die Nukleinsäuresequenz, welche für das TBP kodiert, eine Comutation aufweisen. Durch diese Comutation bindet das TBP an die mutierte TATA-Box (z. B. an TGTATAA) und führt damit zur effizienten Transkription des Effektorgenes. Derartige Comutationen des TBP-Genes sind beispielsweise von Strubin und Struhl (Cell, 721 (1992)) und von Heard et al. (EMBO J., 3519 (1993)) beschrieben worden.

### 7.3.) Multiple Promotoren in Kombination mit einem nuklearen Retentions-Signal und einem nuklearen Export-Faktor

Diese Technologie ist in der Patentanmeldung DE19617851.7 bereits ausführlich beschrieben worden. Auf diese Patentanmeldung wird Bezug genommen.

Ein derartiger Promotor enthält erfindungsgemäß folgende Komponenten:
- eine erste endothelzellspezifische, aktivierbare Promotor- oder Enhancersequenz, die die basale Transkription eines Transgens aktiviert
- ein Transgen, das als Effektorgen für einen Wirkstoff kodiert
- ein nukleares Retentions-Signal (NRS), dessen cDNA am 5'-Ende mit dem 3'-Ende des Strukturgenes (b) mittelbar oder unmittelbar verknüpft ist.
- Bevorzugterweise hat das Transkriptionsprodukt des nuklearen Retentions-Signals eine Bindestruktur für einen nuklearen Export-Faktor.
- Eine weitere unspezifische, zellspezifische, virusspezifische, metabolisch und/oder zellzyklusspezifisch aktivierbare Promotor- oder Enhancersequenz, welche die basale Transkription eines nuklearen Export-Faktors aktiviert
- eine Nukleinsäure kodierend für einen nuklearen Export-Faktor (NEF), der an das Transkriptionsprodukt des nuklearen Retentions-Signals bindet und hierdurch den Transport des Transkriptionsproduktes des Transgenes aus dem Zellkern vermittelt.

Bevorzugt wird das Gen kodierend für das nukleare Retentions-Signal ausgewählt aus der Gruppe umfassend das Rev- responsive Element (RRE) von HIV-1 oder HIV-2, das RRE-äquivalente Retentions-Signal von Retroviren oder das RRE-äquivalente Retentions-Signal des HBV.

Der nukleare Export-Faktor ist bevorzugterweise ein Gen ausgewählt aus der Gruppe umfassend das Rev-Gen der Viren HIV-1, HIV-2, Maedi-Visna Virus, Caprine arthritis encephalitis Virus, das Virus der infektiösen Anämie des Pferdes, das Immundefizienzvirus der Katze, von Retroviren, von HTLV oder das Gen des hnRNP-A1-Proteins oder das Gen des Tanskriptionsfaktors TFIII-A.

### 7.4.) Aktivator-responsive Promotoreinheit

Aktivator-responsive Promotoreinheiten sind in der Patentanmeldung DE19617851.7 bereits im Detail beschrieben worden. Auf diese Patentanmeldung wird Bezug genommen.

Eine Aktivator-responsive Promotoreinheit besteht aus folgenden Komponenten:
- eine oder mehrere gleiche oder unterschiedliche Promotor- oder Enhancersequenz(en), die beispielsweise zellzyklusspezifisch, zellproliferationsabhängig, metabolisch, endothelzellspezifisch oder virusspezifisch oder sowohl zellzyklusspezifisch als auch metabolisch, endothelzellspezifisch oder virusspeziflsch (sogenannte chimäre Promotoren) aktivierbar ist bzw. sind
- eine oder mehrere gleiche oder unterschiedliche Aktivatorsubeinheit(en), welche jeweils stromabwärts von den Promotor- oder Enhancersequenzen gelegen und durch diese in ihrer basalen Transkription aktiviert wird bzw. werden
- einen Aktivator-responsiven Promotor, welcher durch die Expressionsprodukte von einer oder mehreren Aktivatorsubeinheit(en) aktiviert wird.

In einer bevorzugten Ausführungsform können erfindungsgemäße Aktivatorresponsive Promotoreinheiten Bindesequenzen für chimäre Transkriptionsfaktoren aus DNA-Bindungsdomänen, Protein-Proteininteraktionsdomänen und Transaktivietungsdomänen darstellen. Alle in der Anmeldung genannten Transkriptionsfaktorbindungsstellen können einfach (Monomere) oder in mehreren Kopien (Multimere beispielsweise bis zu 10 Kopien) vorliegen.

Ein Beispiel für einen Aktivator-responsiven Promotor aktiviert durch zwei Aktivatorsubeinheiten stellt der LexA-Operator in Verbindung mit dem SV40-Promotor dar.
- Die erste Aktivatorsubeinheit umfaßt die cDNA für das LexA-DNA-Bindeprotein kodierend für die Aminosäuren 1-81 oder 1-202, deren 3'-Ende verknüpft ist mit dem 5'-Ende der cDNA für das Gal80-Protein (Aminosäuren 1-435).
- Die zweiter Aktivatorsubeinheit umfaßt die cDNA der Gal80-Bindungsdomäne des Gal4-Proteins kodierend für die Aminosäuren 851-881, deren 3'-Ende verknüpft ist mit dem 5'-Ende der cDNA des SV40 large T-Antigens kodierend für die Aminosäuren 126-132, deren 3'-Ende verknüpft ist mit dem 5'-Ende der cDNA für die Transaktivierungsdomäne des VP16 von HSV-1 kodierend für die Aminosäuren 406-488.

Ein weiteres Beispiel für einen Aktivator-responsiven Promotor aktiviert durch zwei Aktivatorsubeinheiten stellt die Bindesequenz für das Gal4-Protein in Verbindung mit dem SV40-Promotor dar.
- Die erste Aktivierungseinheit umfaßt die cDNA für die DNA-Bindedomäne des Gal4-Proteins (Aminosäuren 1-147), deren 3'-Ende verknüpft ist mit dem 5'-Ende der cDNA für das Gal80-Protein (Aminosäuren 1-435).
- Die zweite Aktivierungssubeinheit umfaßt die cDNA für die Gal80-Bindungsdomäne von Gal4 (Aminosäuren 851-881), deren 3'-Ende verknüpft ist mit dem 5'-Ende der cDNA des nuklearen Lokalisationssignals von SV40 (SV40 large T; Aminosäuren 126-132), deren 3'-Ende verknüpft ist mit dem 5'-Ende der cDNA für die Transaktivierungsdomäne des VP16 von HSV-1 kodierend für die Aminosäuren 406-488.

Ein weiteres Beispiel für zwei Aktivatorsubeinheiten, die den Aktivator-responsiven Promotor, bestehend aus der Bindesequenz für das Gal4-Protein und dem SV40-Promotor, aktivieren, stellt dar
- eine erste Aktivierungseinheit, die die cDNA für die zytoplasmische Domäne des CD4-T-Zellantigens (Aminosäuren 397-435) umfaßt, deren 5'-Ende verknüpft ist mit dem 3'-Ende der cDNA für die Transaktivierungsdomäne des VP16 von HSV-1 (Aminosäuren 406-488), deren 5'-Ende wiederum verknüpft ist mit dem 3'-Ende der cDNA des nuklearen Lokalisationssignals von SV40 (SV40 large T; Aminosäuren 16-132) und
- die zweiter Aktivierungseinheit umfassend die cDNA des nuklearen Lokalisationssignals von SV40 (SV40 large T; Aminosäuren 126-132), die cDNA für die DNA Bindedomäne des Gal4-Proteins (Aminosäuren 1-147), deren 3'-Ende verknüpft ist mit dem 5'-Ende der cDNA für die CD4 Bindesequenz des p56 Ick Proteins (Aminosäuren 1-71).

### 8) Auswahl des Effektorgenes

Im Sinne der Erfindung enthält die erfindungsgemäße Nukleotidsequenz mindestens ein Effektorgen (Komponente e), welches für einen pharmakologisch aktiven Wirkstoff kodiert zur Prophylaxe und/oder Therapie einer Erkrankung. Dieser Wirkstoff ist ausgewählt aus einer Gruppe umfassend Cytokine, Wachstumsfaktoren, Antikörper oder Antikörperfragmente, Rezeptoren für Cytokine oder Wachstumsfaktoren, antiproliferativ, apoptotisch oder zytostatisch wirkende Proteine, Angiogeneseinhibitoren, Gerinnungshemmer, fibrinolytisch wirkende Substanzen, Plasmaproteine, Komplement-aktivierende Proteine, Peptidhormone, Virushüllproteine, bakterielle Antigene und parasitäre Antigene, auf den Blutkreislauf wirkende Proteine und Ribozyme.

Bevorzugterweise handelt es sich bei dem Transgen um ein Strukturgen, das für ein Ribozym kodiert, welches die mRNA inaktiviert, welche kodiert für ein Protein ausgewählt aus der Gruppe umfassend Zellzykluskontrollproteine, insbesondere Cyclin A, Cyclin B, Cyclin D1, Cyclin E, E2F1-5, cdc2, cdc25C oder DP1 oder Virusproteine oder Cytokine oder Wachstumsfaktoren oder deren Rezeptoren. In einer weiteren Ausführungsform kann das Effektorgen für ein Enzym kodieren, welches eine Vorstufe eines Pharmakons in ein Pharmakon spaltet.

In einer weiteren Ausführungsform kann das Effektorgen für ein Ligand-Effektorfusionsprotein kodieren, wobei der Ligand ein Antikörper, ein Antikörperfragment, ein Cytokin, ein Wachstumsfaktor, ein Adhäsionsmolekül oder ein Peptidhormon sein kann und der Effektor ein wie oben beschriebener pharmakologisch aktiver Wirkstoff oder ein Enzym. Beispielsweise kann das Strukturgen ein Ligand-Enzymfusionsprotein kodieren, wobei das Enzym eine Vorstufe eines Pharmakons in ein Pharmakon spaltet und der Ligand an eine Zelloberfläche bindet, bevorzugt an Endothelzellen oder Tumorzellen.

Im Sinne der Erfindung richtet sich die Auswahl des Effektorgenes und des ggf. mit dem endothelzellspezifischen Promotor zu kombinierenden weiteren Promotorelementes nach Art der Prophylaxe und/oder Therapie der jeweiligen Erkrankung.

Beispielsweise sind bei folgenden Erkrankungen folgende Kombinationen von Promotorsequenzen und Effektorgenen zu wählen (eine detaillierte Beschreibung erfolgte bereits in den Patentanmeldungen EP97101507.8; EP97102547.3; DE19710643.9; DE197704301.1; DE19617851.7; DE19639103.2; DE19651443.6; EP95931204.2; EP95930524.4; EP95931205.9; EP95931933.6 und DE19701141.1, auf die Bezug genommen wird).

### 8.1.) Therapie von Tumoren

### 8.1.1.) zusätzliche Promotoren:

- unspezifisch und/oder
- zellzyklusspezifisch und/oder
- metabolisch aktivierbar

### 8.1.2.) Effektorgene für Inhibitoren der Zellproliferation, zum Beispiel für

- das Retinoblastomprotein (pRb=p110) oder die verwandten p107 und p130 Proteine
- Das Retinoblastomprotein (pRb/p110) und die verwandten p107 und p130 Proteine werden durch Phosphorylierung inaktiviert. Bevorzugt sind solche Gene dieser Zellzyklusinhibitoren zu verwenden, welche Mutationen für die Inaktivierungsstellen der exprimierten Proteine aufweisen, ohne daß diese hierdurch in ihrer Funktion beeinträchtigt werden. Beispiele für diese Mutationen wurden beschrieben für das p110. In analoger Weise wird die DNA-Sequenz für das p107 Protein oder das p130 Protein mutiert.
- das p53 Protein
- Das Protein p53 wird in der Zelle inaktiviert entweder durch Bindung an spezielle Proteine, wie beispielsweise MDM2, oder durch Oligomerisierung des p53 über das dephosphorylierte C-terminale Serin. Bevorzugt wird somit eine DNA-Sequenz für ein p53 Protein verwendet, welches C-terminal verkürzt ist um das Serin 392.
- das p21 (WAF-1)
- das p16 Protein
- andere cdk-Inhibitoren
- das GADD45 Protein
- das bak Protein

### 8.1.3.) Effektorgene für Gerinnung induzierende Faktoren und Angiogeneseinhibitoren, zum Beispiel:

- Plasminogenaktivatorinhibitor-1 (PAI-1)
- PAI-2
- PAI-3
- Angiostatin
- Interferone (IFNα, IFNβ oder IFNγ)
- Platelet factor 4
- TIMP-1
- TIMP-2
- TIMP-3
- Leukemia Inhibitory Factor (LIF)
- Tissue Factor (TF) und dessen gerinnungsaktive Fragmente
- Faktor X oder Mutationen von Faktor X entsprechend der Patentanmeldung D19701141.1, auf welche ausdrücklich Bezug genommen wird.

### 8.1.4.) Effektorgene für zytostatische und zytotoxische Proteine, zum Beispiel für

- Perforin
- Granzym
- IL-2
- IL-4
- IL-12
- Interferone, wie beispielsweise IFN-α, IFNβ oder IFNγ
- TNF, wie TNFα oder TNFβ
- Oncostatin M
- Sphingomyelinase
- Magainin und Magainin-Derivate

### 8.1.5.) Effektorgene für zytostatische oder zytotoxische Antikörper und für Fusionsproteine zwischen antigenbindenden Antikörperfragmenten mit zytostatischen, zytotoxischen oder entzündungserregenden Proteinen oder Enzymen.

- Zu den zytostatischen oder zytotoxischen Antikörpern gehören solche gerichtet gegen Membranstrukturen von Endothelzellen wie sie beispielsweise von Burrows et al. (Pharmac. Ther 64, 155 (1994)), Hughes et al., (Cancer Res. 49, 6214 (1989)) und Maruyama et al., (PNAS USA 87, 5744 (1990)) beschrieben wurden. Insbesondere zählen hierzu Antikörper gegen die VEGF-Rezeptoren.
- Des weiteren gehören hierzu zytostatische oder zytotoxische Antikörper gerichtet gegen Membranstrukturen auf Tumorzellen. Derartige Antikörper wurden zum Beispiel von Sedlacek et al., Contrib. to Oncol. 32, Karger Verlag, München (1988) und Contrib. to Oncol. 43, Karger Verlag, München (1992) übersichtlich dargestellt. Weitere Beispiele stellen dar Antikörper gegen Sialyl Lewis; gegen Peptide auf Tumoren, welche von T-Zellen erkannt werden; gegen von Onkogenen exprimierte Proteine; gegen Ganglioside wie GD3, GD2, GM2, 9-O-acetyl GD3, Fucosyl GM1; gegen Blutgruppenantigene und deren Vorläufer; gegen Antigene auf dem polymorphen epithelialen Mucin; gegen Antigene auf Heat Shock Proteinen
- Des weiteren gehören hierzu gehören Antikörper gerichtet gegen Membranstrukturen von Leukämiezellen. Eine große Anzahl derartiger monoklonaler Antikörper sind bereits für diagnostische und therapeutische Verfahren beschrieben worden (Übersichten bei Kristensen, Danish Medical Bulletin 41, 52 (1994); Schranz, Therapia Hungarica 38, 3 (1990); Drexler et al., Leuk. Res. 10, 279 (1986); Naeim, Dis. Markers 7, 1 (1989); Stickney et al., Curr. Opin. Oncol. 4, 847 (1992); Drexler et al., Blut 57, 327 (1988); Freedman et al., Cancer Invest 9, 69 (1991)). Je nach Typ der Leukämie sind als Liganden beispielsweise monoklonalen Antikörper oder deren antigenbindende Antikörperfragmente gerichtet gegen folgende Membranantigene geeignet: 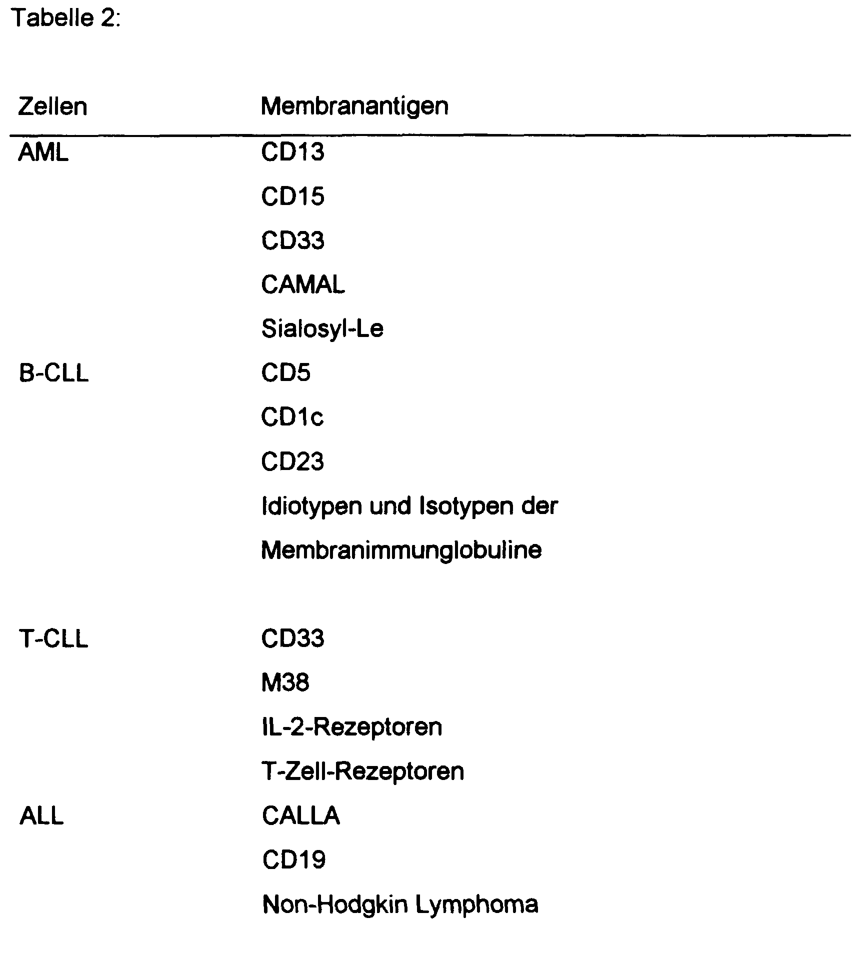
- Die Humanisierung muriner Antikörper, die Herstellung und Optimierung der Gene für Fab und rek. Fv Fragmente erfolgt entsprechend der dem Fachmann bekannten Technik (Winter et al., Nature 349, 293 (1991); Hoogenbooms et al., Rev. Tr. Transfus. Hemobiol. 36, 19 (1993); Girol. Mol. Immunol. 28, 1379 (1991) oder Huston et al., Intern. Rev. Immunol. 10, 195 (1993)). Die Fusion der rek. Fv-Fragmente mit Genen für zytostatische, zytotoxische oder entzündungserregende Proteinen oder Enzymen erfolgt gleichermaßen entsprechend dem dem Fachmann bekannten Stand der Technik.

### 8.1.6.) Effektorgene für Fusionsproteine von weiteren Endothelzell- oder Tumorzellbindenden Liganden mit zytostatischen und zytotoxischen Proteinen oder Enzymen. Zu den Liganden gehören zum Beispiel alle Substanzen, welche an Membranstrukturen oder Membranrezeptoren auf Endothelzellen binden. Beispielsweise gehören hierzu

- Antikörper oder Antikörperfragmente
- Cytokine wie beispielsweise IL-1 oder Wachstumsfaktoren oder deren Fragmente bzw. Teilsequenzen von ihnen, die an Rezeptoren exprimiert durch Endothelzellen binden, wie beispielsweise PDGF, bFGF, VEGF, TGF.
- Des weiteren gehören hierzu Adhäsionsmoleküle, welche an aktivierte und/oder proliferierende Endothelzellen binden. Hierzu gehören beispielsweise SLex, LFA-1 , MAC-1, LECAM-1, VLA-4 oder Vitronectin.
- Hierzu gehören des weiteren Substanzen, welche an Membranstrukturen oder Membranrezeptoren von Tumor- oder Leukämiezellen binden. Beispielsweise gehören hierzu Wachstumsfaktoren oder deren Fragmente bzw. Teilsequenzen von ihnen, die an Rezeptoren exprimiert durch Leukämiezellen oder Tumorzellen binden.
   Derartige Wachstumsfaktoren wurden bereits beschrieben (Übersichten bei Cross et al., Cell 64, 271 (1991), Aulitzky et al., Drugs 48, 867 (1994), Moore, Clin. Cancer Res. 1, 3 (1995), Van Kooten et al., Leuk. Lymph. 12, 27 (1993)).
- Die Fusion der Gene dieser an die Zielzelle bindenden Liganden mit zytostatischen, zytotoxischen oder entzündungserregenden Proteinen oder Enzymen erfolgt entsprechend dem Stand der Technik mit den dem Fachmann bekannten Methoden.

### 8.1.7.) Effektorgene für Induktoren von Entzündungen, zum Beispiel für

- IL-1
- IL-2
- RANTES (MCP-2)
- monocyte chemotactic and activating factor (MCAF)
- IL-8
- macrophage inflammatory protein-1 (MIP-1α, -β)
- neutrophil activating protein-2 (NAP-2)
- IL-3
- IL-5
- human leukemia inhibitory factor (LIF)
- IL-7
- IL-5
- Eotaxin
- IL-13
- GM-CSF
- G-CSF
- M-CSF
- Cobra venom factor (CVF) oder Teilsequenzen vom CVF, welchem dem menschlichen Komplementfaktor C3b funktionell entsprechen, d. h. welche an den Komplementfaktor B binden können und nach Spaltung durch den Faktor D eine C3 Konvertase darstellen
- der menschliche Komplementfaktor C3 oder seine Teilsequenz C3b
- Spaltprodukte des menschlichen Komplemenffaktors C3, welche funktionell und strukturell dem CVF ähneln
- bakterielle Proteine, welche Komplement aktivieren oder Entzündungen auslösen, wie beispielsweise Porine von Salmonella typhimurium, "clumping" Faktoren von Staphylococcus aureus, Moduline besonders von gram-negativen Bakterien, "Major outer membrane protein" von Legionellen oder von Haemophilus influenza Typ B oder von Klebsiellen oder M-Moleküle von Streptokokken Gruppe G.

### 8.1.8.) Effektorgene für Enzyme für die Aktivierung von Vorstufen von Zytostatika, zum Beispiel für Enzyme, welche inaktive Vorsubstanzen (Prodrings) in aktive Zytostatika (Drugs) spalten.

Derartige Substanzen und die jeweils zugehörigen Prodrugs und Drugs sind bereits von Deonarain et al. (Br. J. Cancer 70, 786 (1994)), Mullen, (Pharmac. Ther. 63, 199 (1994)) und Harris et al. (Gene Ther. 1, 170 (1994)) übersichtlich beschrieben worden. Beispielsweise ist die DNA-Sequenz eines der folgenden Enzyme zu verwenden:
- Herpes Simplex Virus thymidinkinase
- Varizella Zoster Virus Thymidinkinase
- bakterielle Nitroreduktase
- bakterielle β-Glucuronidase
- pflanzliche β-Glucuronidase aus Secale cereale
- humane β-Glucuronidase
- humane Carboxypeptidase (CB) zum Beispiel CB-A der Mastzelle, CB-B des Pankreas oder bakterielle Carboxy peptidase
- bakterielle β-Laktamase
- bakterielle Cytosine deaminase
- humane Catalase bzw. Peroxidase
- Phosphatase, im besonderen humane alkalische Phosphatase, humane saure Prostataphosphatase oder Typ 5 saure Phosphatase
- Oxidase, im besonderen humane Lysyloxidase oder humane saure D-aminooxidase
- Peroxidase, im besonderen humane Glutathion Peroxidase, humane Eosinophilen Peroxidase oder humane Schilddrüsen Peroxidase
- Galaktosidase

### 8.2.) Therapie von Autoimmunerkrankungen und Entzündungen

### 8.2.1.) zusätzliche Promotoren:

- unspezifisch und/oder
- zellzyklusspezifisch und/oder
- metabolisch aktivierbar

### 8.2.2.) Effektorgene für die Therapie von Allergien, zum Beispiel für

- IFNβ
- IFNγ
- IL-10
- Antikörper bzw. Antikörperfragmente gegen IL-4
- lösliche IL-4-Rezeptoren
- IL-12
- TGFβ

### 8.2.3.) Effektorgene für die Verhinderung der Abstoßung von transplantierten Organen, zum Beispiel für

- IL-10
- TGFβ
- lösliche IL-1-Rezeptoren
- lösliche IL-2-Rezeptoren
- IL-1-Rezeptorantagonisten
- lösliche IL-6-Rezeptoren
- immunsuppressive Antikörper oder deren V_{H}- und V_{L}- enthaltende Fragmente oder deren über einen Linker verbundene V_{H}- und V_{L}-Fragmente. Immunsuppressive Antikörper sind beispielsweise Antikörper spezifisch für den T-Zell-Rezeptor oder seinen CD3-Komplex, gegen CD4 oder CD8 des weiteren gegen den IL-2-Rezeptor, IL-1-Rezeptor oder IL-4-Rezeptor oder gegen die Adhäsionsmoleküle CD2, LFA-1, CD28 oder CD40

### 8.2.4.) Effektorgene für die Therapie von Antikörper-mediierten Autoimmunerkrankungen, zum Beispiel für

- TGFβ
- IFNα
- IFNβ
- IFNγ
- IL-12
- lösliche IL-4-Rezeptoren
- lösliche IL-6-Rezeptoren
- immunsuppressive Antikörper oder dessen V_{H}- und V_{L}-enthaltende Fragmente

### 8.2.5.) Effektorgene für die Therapie von Zell-mediierten Autoimmunerkrankungen, zum Beispiel für

- IL-6
- IL-9
- IL-10
- IL-13
- TNFα oder TNFβ
- einen immunsuppressiven Antikörper oder dessen V_{H}- und V_{L}-enthaltende Fragmente

### 8.2.6.) Effektorgene für Inhibitoren der Zellproliferation, zytostatische oder zytotoxische Proteine, Induktoren von Entzündungen und Enzyme für die Aktivierung von Vorstufen von Zytostatika

Beispiele für Gene kodierend für derartige Proteine sind bereits im Abschnitt "Strukturgene für die Therapie von Tumoren" aufgeführt.

In gleicher Form wie dort bereits beschrieben, können im Sinne der Erfindung Strukturgene verwendet werden, welche für Fusionsproteine aus Antikörpern bzw. Fab oder rek. Fv-Fragmenten dieser Antikörper oder anderen Liganden spezifisch für die Zielzelle und den o. a. Cytokinen, Wachstumsfaktoren, Rezeptoren, zytostatischen oder zytotoxischen Proteinen und Enzymen kodieren.

### 8.2.7.) Strukturgene für die Therapie der Arthritis

Im Sinne der Erfindung werden Strukturgene ausgewählt, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

Hierzu gehören zum Beispiel
- IL-1-Rezeptorantagonist (IL-1-RA);
   IL-1-RA inhibiert die Bindung von IL-1α, β
- löslicher IL-1-Rezeptor;
   löslicher IL-1-Rezeptor bindet und inaktiviert IL-1
- IL-6
   IL-6 erhöht die Sekretion von TIMP und Superoxiden und vermindert die Sekretion von IL-1 und TNFα durch Synovialzellen und Chondrozyten
- löslicher TNF-Rezeptor
   löslicher TNF-Rezeptor bindet und inaktiviert TNF.
- IL-4
   IL-4 inhibiert die Bildung und Sekretion von IL-1, TNFα und MMP
- IL-10
   IL-10 inhibiert die Bildung und Sekretion von IL-1, TNFα und MMP und erhöht die Sekretion von TIMP
- Insulin-like growth factor (IGF-1)
   IGF-1 stimuliert die Synthese von extrazellulärer Matrix.
- TGFβ, im speziellen TGFβ1 und TGFβ2
   TGFβ stimuliert die Synthese von extrazellulärer Matrix.
- Superoxiddismutase
- TIMP, im speziellen TIMP-1, TIMP-2 oder TIMP-3

### 8.3.) Therapie der mangelhaften Bildung von Zellen des Blutes

### 8.3.1.) zusätzliche Promotoren

- zellunspezifisch und/oder
- zellzyklusspezifisch und/oder
- metabolisch aktivierbar

### 8.3.2.) Effektorgene für die Therapie der Anämie, zum Beispiel für

- Erythropoietin

### 8.3.3.) Effektorgene für die Therapie der Leukopenie, zum Beispiel für

- G-CSF
- GM-CSF
- M-CSF

### 8.3.4.) Effektorgene für die Therapie der Thrombozytopenie, zum Beispiel für

- IL-3
- Leukemia Inhibitory Factor (LIF)
- IL-11
- Thrombopoietin

### 8.4.) Therapie von Schäden des Nervensystems

### 8.4.1.) zusätzliche Promotoren

- unspezifisch und/oder
- zellzyklusspezifisch und/oder
- metabolisch aktivierbar

### 8.4.2.) Effektorgene für neuronale Wachstumsfaktoren, zum Beispiel

- FGF
- Nerve growth factor (NGF) Brain-derived neurotrophic factor (BDNF)
- Neurotrophin-3 (NT-3)
- Neurotrophin-4 (NT-4)
- Ciliary neurotrophic factor (CNTF)

### 8.4.3.) Effektorgene für Enzyme, zum Beispiel für

- Tyrosinhydroxylase
- Dopadecarboxylase

### 8.4.4.) Effektorgene für Cytokine und deren Inhibitoren, welche die neurotoxische Wirkung von TNFα inhibieren oder neutralisieren, zum Beispiel für

- TGFβ
- lösliche TNF-Rezeptoren
   TNF-Rezeptoren neutralisieren TNFα
- IL-10
   IL-10 inhibiert die Bildung von IFNγ, TNFα, IL-2 und IL-4
- lösliche IL-1-Rezeptoren
- IL-1-Rezeptor I
- IL-1-Rezeptor
   lösliche IL-1-Rezeptoren neutralisieren die Aktivität von IL-1
- IL-1-Rezeptor-Antagonist
- lösliche IL-6-Rezeptoren

### 8.5.) Therapie von Störungen des Blutgerinnungs- und Blutkreislaufsystems

### 8.5.1.) zusätzliche Promotoren

- zellzyklusspezifisch und/oder
- zellunspezifisch und/oder
- metabolisch aktivierbar

### 8.5.2.) Effektorgene für die Inhibition der Gerinnung oder für die Förderung der Fibrinolyse, zum Beispiel für

- Tissue Plasminogen Activator (tPA)
- Urokinase-type Plasminogen Activator (uPA)
- Hybride von tPA und uPA
- Protein C
- Hirudin
- Serin Proteinase Inhibitoren (Serpine), wie beispielsweise C-1S-Inhibitor, α1-Antitrypsin oder Antithrombin III
- Tissue Factor Pathway Inhibitor (TFPI)

### 8.5.3.) Effektorgene für die Förderung der Gerinnung, zum Beispiel für

- F VIII
- F IX
- von Willebrand factor
- F XIII
- PAI-1
- PAI-2
- Tissue Factor und Fragmente hiervon

### 8.5.4.) Effektorgene für Angiogenesefaktoren, zum Beispiel für

- VEGF
- FGF

### 8.5.5.) Effektorgene für die Blutdrucksenkung, zum Beispiel für

- Kallikrein
- Endothelzell "nitric oxide synthase"

### 8.5.6.) Effektorgene für die Inhibition der Proliferation von glatten Muskelzellen nach Verletzungen der Endothelschicht, zum Beispiel für

- ein antiproliferatives, zytostatisches oder zytotoxisches Protein oder
- ein Enzym zur Aufspaltung von Vorstufen von Zytostatika in Zytostatika wie bereits oben (unter Tumor) aufgeführt oder
- ein Fusionsprotein eines dieser Wirkstoffe mit einem Liganden, beispielsweise einem Antikörper oder Antikörperfragmenten spezifisch für Muskelzellen

### 8.5.7.) Effektorgene für weitere Blutplasmaproteine, zum Beispiel für

- Albumin
- C1-Inaktivator
- Serum Cholinesterase
- Transferrin
- 1-Antritrypsin

### 8.6.) Impfungen

### 8.6.1.) zusätzliche Promotoren

- unspezifisch und/oder
- zellzyklusspezifisch

### 8.6.2.) Effektorgene für die Prophylaxe von Infektionserkrankungen

Die Möglichkeiten, auf konventionellem Wege wirkungsvolle Impfstoffe herzustellen, sind beschränkt.

Demzufolge wurde die Technologie der DNA-Vakzine entwickelt. Diese DNA-Vakzinen werfen jedoch Fragen zur Wirksamkeitsstärke auf (Fynan et al., Int. J. Immunopharm. 17, 79 (1995); Donnelly et al., Immunol. 2, 20 (1994)).

Gemäß dieser Erfindung ist mit einer größeren Wirksamkeit der DNA-Vakzine zu rechnen.

Als Wirksubstanz ist die DNA eines vom Infektionserreger gebildeten Proteins auszuwählen, welches durch Auslösung einer Immunreaktion, d. h. durch Antikörperbindung und/oder durch zytotoxische T-Lymphozyten zur Neutralisierung und/oder zur Abtötung des Erregers führt. Derartige sogenannte Neutralisationsantigene werden als Impfantigene bereits angewandt (siehe Übersicht bei Ellis, Adv. Exp. Med. Biol. 327, 263 (1992)).

Bevorzugt im Sinne der Erfindung wird die DNA kodierend für Neutralisationsantigene folgender Erreger:
- Influenza A-Virus
- HIV
- Tollwut-Virus
- HSV (Herpes Simplex Virus)
- RSV (Respiratory Syncytial Virus)
- Parainfluenza-Virus
- Rotavirus
- VZV (Varizella Zoster Virus)
- CMV (Cytomegalo-Virus)
- Masern-Virus
- HPV (Humanes Papillomvirus)
- HBV (Hepatitis B-Virus)
- HCV (Hepatitis C-Virus)
- HDV (Hepatitis D-Virus)
- HEV (Hepatitis E-Virus)
- HAV (Hepatitis A-Virus)
- Vibrio Cholera-Antigen
- Borrelia burgdorferi
- Helicobacter pylori
- Malaria-Antigen
- Zu derartigen Wirksubstanzen im Sinne der Erfindung gehört jedoch auch die DNA eines Antiidiotyp-Antikörpers oder seiner Antigen-bindenden Fragmente, dessen Antigenbindungsstrukturen (die "complementary determining regions") Kopien der Protein- oder Kohlenhydratstruktur des Neutralisationsantigens des Infektionserregers darstellen.

Derartige Antiidiotyp-Antikörper können besonders Kohlenhydratantigene bei bakteriellen Infektionserregern ersetzen.

Derartige antiidiotypische Antikörper und ihre Spaltprodukte wurden von Hawkins et al. (J. Immunother. 14, 273 (1993)) und Westerink und Apicella (Springer Seminars in Immunopathol. 15, 227 (1993)) übersichtlich beschrieben.

### 8.6.3.) Effektorgene für "Tumorvakzinen"

Hierzu gehören Antigene auf Tumorzellen. Derartige Antigene wurden zum Beispiel von Sedlacek et al., Contrib. to Oncol. 32, Karger Verlag, München (1988) und Contrib. to Oncol 43, Karger Verlag, München (1992) übersichtlich dargestellt.

Weitere Beispiele stellen die Gene für bzw. folgende Antigene bzw. für folgende Antiidiotypantikörper dar:
- Sialyl Lewis
- Peptide auf Tumoren, welche von T-Zellen erkannt werden
- von Onkogenen exprimierte Proteine
- Blutgruppenantigene und deren Vorläufer
- Antigene auf dem polymorphen epithelialen Mucin
- Antigene auf Heat Shock Proteinen

### 8.7.) Die Therapie von chronischen Infektionserkrankungen

### 8.7.1.) zusätzliche Promotoren

- virusspezifisch und/ oder
- zellzyklusspezifisch und/oder
- unspezifisch

### 8.7.2.) Effektorgene, beispielsweise für

- ein Protein, welches zytostatische, apoptotische oder zytotoxische Wirkungen aufweist.
- ein Enzym, welches eine Vorstufe einer antiviralen oder zytotoxischen Substanz in die aktive Substanz spaltet.

### 8.7.3.) Effektorgene für antivirale Proteine

- antiviral wirksame Cytokine und Wachstumsfaktoren. Hierzu zählen beispielsweise IFNα, IFNβ, IFN-γ, TNFβ, TNFα, IL-1 oder TGFβ
- Antikörper einer Spezifität, die das jeweilige Virus inaktiviert oder dessen V_{H} und V_{L} enthaltende Fragmente oder dessen über einen Linker verbundene V_{H} und V_{L} Fragmente herstellt wie bereits beschrieben.
   Antikörper gegen Virusantigen sind beispielsweise:
   anti HBV
   anti HCV
   anti HSV
   anti HPV
   anti HIV
   anti EBV
   anti HTLV
   anti Coxsackie Virus
   anti Hantaan Virus
- ein Rev bindendes Protein. Diese Proteine binden an die Rev-RNA und inhibieren Rev-abhängige posttranskriptionelle Stufen der Retrovirus-Genexpression. Beispiele für Rev-bindende Proteine sind:
   RBP9-27
   RBP1-8U
   RBP1-8D
   Pseudogene von RBP1-8
- für Ribozyme, welche die mRNA von Genen für Zellzykluskontrollproteine oder die mRNA von Viren verdauen. Ribozyme katalytisch für HIV wurden beispielsweise von Christoffersen et al., J. Med. Chem. 38, 2033 (1995) übersichtlich beschrieben.

### 8.7.4.) Effektorgene für antibakterielle Proteine

Zu den antibakteriellen Proteinen gehören beispielsweise Antikörper, die bakterielle Toxine neutralisieren oder Bakterien opsonieren. Beispielsweise gehören hierzu Antikörper gegen
Meningokokken C oder B
coli
Borrelia
Pseudomonas
Helicobacter pylori
Staphylococcus aureus

### 9) Kombination gleicher oder unterschiedlicher Strukturgene

Gegenstand der Erfindung ist des weiteren ein Nukleinsäurekonstrukt, in welchem eine Kombination der DNA-Sequenzen von zwei gleichen oder zwei unterschiedlichen Strukturgenen vorliegt. Zur Expression beider DNA-Sequenzen ist eine weitere Promotorsequenz oder vorzugsweise die cDNA einer "internal ribosome entry site" (IRES) als regulatorisches Element zwischen beiden Stukturgenen geschaltet.

Eine IRES ermöglicht die Expression zweier über eine IRES miteinander verbundener DNA-Sequenzen.

Derartige IRES wurden beispielsweise von Montford und Smith (TIG 11, 179 (1995); Kaufman et al., Nucl. Acids Res. 19, 4485 (1991); Morgan et al., Nucl. Acids Res. 20, 1293 (1992); Dirks et al., Gene 128, 247 (1993); Pelletier und Sonenberg, Nature 334, 320 (1988) und Sugitomo et al., BioTechn. 12, 694 (1994)) beschrieben.

So kann beispielsweise die cDNA der IRES-Sequenz des Poliovirus (Position ≤ 140 bis ≥ 630 des 5' UTR) verwendet werden.

Bevorzugt im Sinne der Erfindung sind über weitere Promotorsequenzen oder eine IRES-Sequenz Strukturgene zu verknüpfen, welche eine additive Wirkung aufweisen.

Im Sinne der Erfindung sind bevorzugte Kombinationen von Strukturgenen beispielsweise für

### 9.1.) die Therapie von Tumoren

- gleiche oder unterschiedliche, zytostatische, apoptotische, zytotoxische oder entzündungserregende Proteine oder
- gleiche oder unterschiedliche Enzyme für die Spaltung der Vorstufe eines Zytostatikums

### 9.2.) die Therapie von Autoimmunerkrankungen

- unterschiedliche Cytokine oder Rezeptoren mit synergistischer Wirkung zur Hemmung der zellulären und/oder humoralen Immunreaktion oder
- unterschiedliche oder gleiche TIMPs

### 9.3.) die Therapie von mangelhafter Bildung von Zellen des Blutes

- unterschiedliche, hierarchisch aufeinanderfolgende Cytokine, wie beispielsweise
   IL-1, IL-3, IL-6 oder GM-CSF und Erythropoietin, G-CSF oder Thrombopoietin

### 9.4.) die Therapie von Nervenzellschäden

- ein neuronaler Wachstumsfaktor und ein Cytokin oder der Inhibitor eines Cytokines

### 9.5.) die Therapie von Störungen des Blutgerinnungs- und Blutkreislaufsystems

- ein Antithrombotikum und ein Fibrinolytikum (z. B. tPA oder uPA) oder
- ein zytostatisches, apoptotisches oder zytotoxisches Protein und ein Antithrombotikum oder ein Fibrinolytikum
- mehrere unterschiedliche, synergistisch wirkende Blutgerinnungsfaktoren, beispielsweise F VIII und vWF oder F VIII und F IX

### 9.6.) Impfungen

- ein Antigen und ein immunstimulierendes Cytokin, wie beispielsweise IL-1α, IL-1β, IL-2, GM-CSF, IL-3 oder IL-4 Rezeptor
- unterschiedliche Antigene eines Infektionserregers oder unterschiedlicher Infektionserreger oder
- unterschiedliche Antigene eines Tumortyps oder unterschiedlicher Tumortypen

### 9.7.) Therapie von viralen Infektionserkrankungen

- ein antivirales Protein und ein zytostatisches, apoptotisches oder zytotoxisches Protein
- Antikörper gegen unterschiedliche Oberflächenantigene eines Virus oder mehrere Viren

### 9.8.) Therapie von bakteriellen Infektionserkrankungen

- Antikörper gegen unterschiedliche Oberflächenantigene und/oder Toxine eines Keimes

### 10) Einfügung von Signalsequenzen und Transmembrandomänen

Eine detaillierte Beschreibung der Technologie erfolgte bereits in den Patentanmeldungen DE19639103.2 und DE19651443.6, auf die ausdrücklich Bezug genommen wird.

### 10.1.) Zur Verstärkung der Translation kann am 3' Ende der Promotorsequenz und unmittelbar am 5' Ende des Startsignals (ATG) der Signal- bzw. Transmembransequenz die Nukleotidsequenz GCCACC oder GCCGCC eingefügt (Kozak, J. Cell Biol. 108, 299 (1989)) werden.

### 10.2.) Zur Erleichterung der Sekretion des Expressionsproduktes des Strukturgenes kann die ggf. in der DNA-Sequenz des Strukturgenes enthaltene homologe Signalsequenz ersetzt werden durch eine heterologe, die intrazelluläre Ausschleusung verbessernde Signalsequenz.

So kann beispielsweise die Signalsequenz für das Immunglobulin (DNA Position ≤ 63 bis ≥ 107; Riechmann et al., Nature 332, 323 (1988)) oder die Signalsequenz für das CEA (DNA-Position ≤ 33 bis ≥ 134; Schrewe et al., Mol. Cell Biol. 10, 2738 (1990); Berling et al., Cancer Res. 50, 6534 (1990)) oder die Signalsequenz des humanen Respiratory Syncytial Virus Glycoproteins (cDNA der Aminosäuren ≤ 38 bis ≥ 50 oder 48 bis 65; Lichtenstein et al., J. Gen. Virol. 77, 109 (1996)) eingefügt werden.

### 10.3.) Zur Verankerung des Wirkstoffes in die Zellmembran der den Wirkstoff bildenden transduzierten Zelle kann alternativ oder zusätzlich zur Signalsequenz eine Sequenz für eine Transmembrandomäne eingeführt werden.

So kann beispielsweise die Transmembransequenz des menschlichen Makrophagenkolonie-stimulierenden Faktors (DNA-Position ≤ 1485 bis ≥ 1554; Cosman et al., Behring Inst. Mitt. 83, 15 (1988)) oder die DNA-Sequenz für die Signal- und Transmembranregion des menschlichen Respiratory Syncytial Virus (RSV)-Glykoproteins G (Aminosäuren 1 bis 63 oder deren Teilsequenzen, Aminosäuren 38 bis 63; Vijaya et al., Mol. Cell Biol. 8, 1709 (1988); Lichtenstein et al., J. Gen. Virol. 77, 109 (1996)) oder die DNA-Sequenz für die Signal- und Transmembranregion der Influenzavirus-Neuraminidase (Aminosäuren 7 bis 35 oder die Teilsequenz Aminosäuren 7 bis 27; Brown et al., J .Virol 62, 3824 (1988)) zwischen der Promotorsequenz und der Sequenz des Strukturgens eingefügt werden.

### 10.4.) Zur Verankerung des Wirkstoffes in die Zellmembran der den Wirkstoff bildenden transduzierten Zellen kann jedoch auch die Nukleotidsequenz für einen Glykophospholipid-Anker eingefügt werden.

Die Einfügung eines Glykophospholipid-Ankers erfolgt am 3' Ende der Nukleotidsequenz für das Strukturgen und kann zusätzlich zur Einfügung einer Signalsequenz erfolgen.

Glykophospholipid-Anker sind beispielsweise für das CEA, für das N-CAM und für weitere Membranproteine, wie beispielsweise Thy-1, beschrieben worden (siehe Übersicht Ferguson et al., Ann. Rev. Biochem. 57, 285 (1988)).

### 10.5.) Eine weitere Möglichkeit der Verankerung von Wirkstoffen an die Zellmembran entsprechend der vorliegenden Erfindung ist die Verwendung einer DNA-Sequenz für ein Ligand-Wirkstoff-Fusionsprotein. Die Spezifität des Liganden dieses Fusionsproteins ist gerichtet gegen eine Membranstruktur auf der Zellmembran der gewählten Zielzelle.

### 10.5.1.) Zu den Liganden, welche an die Oberfläche von Zellen binden, gehören beispielsweise Antikörper oder Antikörperfragmente, gerichtet gegen Strukturen auf der Oberfläche von beispielsweise

- Endothelzellen. Insbesondere zählen hierzu Antikörper gegen die VEGF-Rezeptoren oder gegen Kinin-Rezeptoren
- oder von Muskelzellen, wie Antikörper gegen Actin oder Antikörper gegen Angiotensin II-Rezeptoren oder Antikörper gegen Rezeptoren für Wachstumsfaktoren, wie beispielsweise gegen EGF-Rezeptoren oder gegen PDGF-Rezeptoren oder gegen FGF-Rezeptoren oder Antikörper gegen Endothelin A-Rezeptoren
- Zu den Liganden gehören auch Antikörper oder deren Fragmente, welche gerichtet sind gegen tumorspezifische oder tumorassoziierte Antigene auf der Tumorzellmembran. Derartige Antikörper wurden bereits beschrieben. Die murinen monoklonalen Antikörper sind bevorzugt in humanisierter Form einzusetzen. Fab und rek. Fv-Fragmente und deren Fusionsprodukte werden, wie bereits beschrieben, mit der dem Fachmann bekannten Technologie hergestellt.

### 10.5.2.) Zu den Liganden gehören des weiteren alle Wirkstoffe, wie beispielsweise Cytokine oder Adhäsionsmoleküle, Wachstumsfaktoren oder deren Fragmente bzw. Teilsequenzen von ihnen, Mediatoren oder Peptidhormone, welche an Membranstrukturen oder Membranrezeptoren auf der jeweiligen ausgewählten Zelle binden. Beispielsweise gehören hierzu

- Liganden für Endothelzellen, wie IL-1, PDGF, bFGF, VEGF, TGGβ (Pusztain et al., J. Pathol. 169, 191 (1993)) oder Kinin und Derivate oder Analoga von Kinin.
- Des weiteren gehören hierzu Adhäsionsmoleküle. Derartige Adhäsionsmoleküle, wie beispielsweise SLex, LFA-1, MAC-1, LeCAM-1, VLA-4 oder Vitronectin und Derivate oder Analoga von Vitronectin, wurden bereits für Endothelzellen beschrieben (Übersichten bei Augustin-Voss et al., J. Cell Biol. 119, 483 (1992); Pauli et al., Cancer Metast. Rev. 9, 175 (1990); Honn et al., Cancer Metast. Rev. 11, 353 (1992); Varner et al., Cell Adh. Commun. 3, 367 (1995)).

Die Erfindung wird an folgenden Beispielen näher erläutert, ohne sich darauf zu beschränken.

### 11) Herstellung und Anwendung des Nukleinsäurekonstruktes

Die Nukleinsäurekonstrukte bestehen bevorzugterweise aus DNS. Unter dem Begriff Nukleinsäurekonstrukte werden künstliche Gebilde aus Nukleinsäure verstanden, die in den Zielzellen transkribiert werden können. Sie sind bevorzugt in einen Vektor eingefügt, wobei Plasmidvektoren oder mit nichtviralen Trägern komplexierte Plasmide (Fritz et al., Hum. Gene Ther. 7: 1395 (1996); Solodin et al., Biochem. 34: 13537 (1995); Abdallak et al., Hum Gene Ther. 7: 1947 (1996); Ledley, Hum. Gene Ther. 6: 1129 (1995); Schofield et al., Br. Med. Bull. 51: 56 (1995); Behr, Bioconj. Chem. 5: 382 (1994); Cotten et al., Curr. Opin. Biotechnol. 4: 705 (1993); Hodgson et al., Nature Biotechnol. 14: 339 (1996)) besonders bevorzugt sind. Die Vektoren werden mit den dem Fachmann bekannten Technologien (Cotten et al., Curr. Opin. Biotechnol. 4: 705 (1993); Scheffield et al., Br. Med. Bull. 51: 56 (1995), Ledley, Hum. Gene Ther. 6: 1129 (1995)) in die Vorläuferzelle von Endothelzellen oder in Endothelzellen eingeführt. In einer weiteren Ausführungsform werden die erfindungsgemäßen Nukleinsäurekonstrukte in eine viralen Vektor (Weir et al., Hum. Gene Ther. 7: 1331 (1996); Flotte et al., Gene Ther. 2: 357 (1995); Efstathion et al., Br. Med. Bull. 51: 45 (1995); Kremer et al., Br. Med. Bull. 51: 31 (1995); Vile et al., Br. Med. Bull. 51: 12 (1995); Randrianarison et al., Biologicals 23: 145 (1995); Jolly Cancer Gene Ther. 1: 51 (1994)) eingefügt und mit diesen Endothelzellen transfziert. Die hierdurch transduzierten Zellen werden Patienten äußerlich oder innerlich, lokal, in eine Körperhöhle, in ein Organ, in den Blutkreislauf, in den Atemweg, in den Magen-Darm-Trakt, in den Urogenitaltrakt, in eine Wundhöhle oder intramuskulär oder subkutan verabreicht.

Durch die erfindungsgemäßen Nukleinsäurekonstrukte kann ein Strukturgen in den Endothelzellen oder Vorläuferzellen von Endothelzellen zellspezifisch und ggf. auch virusspezifisch, unter bestimmten metabolischen Bedingungen und/oder zellzyklusspezifisch und/oder induziert durch ein Arzneimittel exprimiert werden, wobei es sich bei dem Strukturgen bevorzugt um ein Gen handelt, das für einen pharmakologisch aktiven Wirkstoff oder aber für ein Enzym kodiert, welches eine inaktive Vorstufe eines Pharmakons in ein aktives Pharmakon spaltet. Das Strukturgen kann so gewählt sein, daß der pharmakologisch aktive Wirkstoff oder das Enzym als Fusionsprotein mit einem Liganden exprimiert wird und dieser Ligand an die Oberfläche von Zellen, z. B. proliferierende Endothel- oder Tumorzellen, bindet.

Die Erfindung wird nun anhand der Figur und der Beispiele näher beschrieben, ohne darauf beschränkt zu sein.

### Figurlegende:

- Fig. 1:: Nukleinsäurekonstrukt zur Transformation von Zellen

### 12) Beispiele zur Erläuterung des Erfindungsgedankens

### 12.1.) Kultivierung von Endothelzellen aus CD34-positiven Blutzellen ohne Verwendung von Fibronektin und Rinderhirn

CD34-positive Blutzellen, isoliert wie von Asahara et al., Science 275: 964 (1997) beschrieben, wurden entweder
- Ansatz a), wie von Asahara publiziert, in Plastikflaschen, beschichtet mit Fibronektin und unter Zugabe von Rinderhirnextrakt (100 µg/ml),
- oder aber (Ansatz b) entsprechend dieser Erfindung in Plastikflaschen ohne Fibronektinbeschichtung und ohne Zugabe von Rinderhirnextrakt, jedoch mit VEGF und bFGF-Zusatz (Fa. Sigma, jeweils 1 % v/v)
   jeweils unter Zusatz von fetalem Kälberserum (FKS, 20 %) in Kulturmedium (Medium 199) bei 37 °C und unter 5 % CO₂-Begasung kultiviert.
   Nach 6 Tagen wurde der Anteil adhäsiv wachsender, spindelförmige und kapillarähnliche Strukturen ausbildender Zellen mikroskopisch und der Anteil an Endothelzellen durch Markierung mit endothelzellspezifischen Antikörpern (anti CD31, anti vWF, anti Flik 1) mit Hilfe der FACS-Analyse ermittelt. Kein Unterschied in der Zahl und Morphologie dieser Zellen wurde zwischen Ansatz a) und Ansatz b) gefunden. In Versuchsserien beider Ansätze schwankte der Anteil von Endothelzellen zwischen 1 und 10 %, was belegt, daß die Zugabe der Wachstumsfaktoren VEGF und bFGF die Beschichtung der Kulturflasche mit Fibronektin und die Zugabe von Hirnextrakt ersetzen kann.

### 12.2.) Kultivierung von Endothelzellen aus mononukleären Zellen des Blutes

Aus 120 ml Blut wurden mit Hilfe der Zentrifugation über einen Ficollgradienten die mononukleären Zellen isoliert und durch Inkubation über 1 Stunde in der Zellkulturflasche und nachfolgender Dekantierung die nicht adhärenten mononukleären Blutzellen abgetrennt.

Diese Zellen wurden in Kulturflaschen entsprechend Ansatz b) ausgesät und über 6 Tage bei 37 °C und 5 %iger CO₂-Begasung kultiviert.

Nach 6 Tagen wurde der Anteil an Endothelzellen, wie unter 12.1) beschrieben, ermittelt. Er lag bei unterschiedlichen Versuchsserien zwischen 2 und 20 %.

### 12.3.) Kultivierung von Endothelzellen aus CD14-positiven Blutzellen

Aus 120 ml Blut eines gesunden Spenders wurden mit Hilfe der Zentrifugation über einen Ficollgradienten (Ficoll-Paque, Pharmacia, Uppsala) die mononukleären Zellen isoliert und durch Inkubation über 60 min in der Zellkulturflasche und nachfolgender Dekantierung die nicht adhärenten mononukleären Blutzellen abgetrennt. Die so isolierten, nicht adhärenten mononukleären Zellen (NMZ) enthielten 0,3 - 0,05 % CD34-positive und 5 - 10 % CD14-positive (Monozyten und monozytenähnliche Zellen) Zellen.

1 x 10⁶ NMZ wurden auf 1 x 10⁶ Zellen/ml Medium 199 enthaltend 20 % fetales Kälberserum (beide von Gibco) und 100 µg ECGS (Harbor Bioproducts, Norwood, MA) oder VEGF (Pepro Techn., London, England) eingestellt und für 1 - 3 Stunden auf Fibronektin (Harbor Bioproducts, Norwood, MA) beschichtete Plastikgefäße bei 37 °C inkubiert. Durch diese Inkubation erhöhte sich der Anteil CD14-positive Zellen von 5 - 10 % auf 25 - 30 %.

Die NMZ wurden durch vorsichtiges Waschen abgetrennt und die CD14-positiven oder CD11-positiven Zellen mit magnetischen Kügelchen, beschichtet mit anti CD14 bzw. anti CD11 (CD14/CD11 Micro Beads, Miltenyi Biotec, Bergisch-Gladbach, Deutschland) entsprechend der Angabe des Herstellers isoliert.
NMZ enthaltend ca. ≥ 80 % CD14-positive Zellen wurden in dem oben angeführten Medium 199, ergänzt mit FKS und ECGS oder VEGF, in Fibronektion beschichteten Plastikgefäßen bei 37 °C mit 5 % CO₂ in angefeuchteter Atmosphäre inkubiert.

Die Zellen in der Kultur wurden nach 6 Stunden, 3 Tagen und 5 Tagen mit Hilfe von monoklonalen Antikörpern und mit Hilfe der RT-PCR untersucht.

Nach 6 Stunden wurden bereits kleine mononukleäre CD14-positive Zellen beobachtet, welche positiv waren für die endothelzellenspezifischen Marker acetyl LDL-Rezeptoren, CD34, Flk-1 und von Willebrand Faktor. Am 3. Tag zeigten diese Zellen starke Anzeichen der Proliferation.

Am Tag 5 waren adhärente große granuläre ovale Zellen und Spindelzellen zu beobachten, die alle aufgeführten Endothelzellmarker trugen jedoch nicht mehr den CD14-Marker.

Sobald diese Endothelzellen zusammenwuchsen, exprimierten sie zusätzlich VE-Cadherin. Nach 1 bis 2 Wochen waren > 80 % der Zellen in der Kultur Endothelzellen.

### 12.4.) Endothelzellspeziflsche Transformation und Kultur von Endothelzellen aus mononukleären Zellen des Blutes

Aus 120 ml Blut wurden mit Hilfe der Zentrifugation über einen Ficollgradienten die mononukleären Zellen isoliert und durch Inkubation über 1 Stunde in der Zellkulturflasche und nachfolgender Dekantierung die nicht adhärenten mononukleären Blutzellen abgetrennt. Diese Blutzellen werden auf eine Konzentration von 1x10⁷/ml Kulturmedium eingestellt, in 60 mm Kulturschalen eingesät und mit einem Komplex aus dem erfindungsgemäßen Plasmid und Superfect (Fa. Quiagen) für 10 min bei 37 °C inkubiert.

Die Herstellung des Komplexes erfolgt nach Angabe des Herstellers von Superfect. Das erfindungsgemäße Plasmid enthält in Leserichtung von 5' nach 3' folgende DNA-Sequenzen:
- der Promotor des humanen Endoglingenes (NS1-2415; Patentanmeldung D19704301.1)
- die cDNA der humanen Cyclin-dependent kinase 4 (cdk-4) mit einer Mutation im Codon 24 [Austausch eines Arginins (CGT) durch ein Cystein (TGT), Wölfel et al. Science 269: 1281 (1997)].
- Das nukleäre Lokalisationssignal (NLS) von SV40 [SV40 large T; Aminosauren 126 bis 132; PKKKRKV (SEQ ID NO.: 3); Dingwall et al., TIBS 16: 478 (1991)].

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzymen und DNA-Ligasen. Diese Enzyme sind käuflich zu erwerben. Mit Hilfe dieser Enzyme wird das so hergestellte Nukleotid einkloniert.

Nach Inkubation der mononukleären Blutzellen mit dem Superfect/Plasmid Komplex werden die Blutzellen gewaschen und in Zellkulturmedium kultiviert wie im Abschnitt 12.2) beschrieben.

Nach 6 Tagen wird der Anteil an Endothelzellen wie im Abschnitt 12.1.) beschrieben bestimmt. Er schwankt bei unterschiedlichen Versuchsserien zwischen 10 und 60 %.

### 12.5.) Herstellung und Verwendung von transduzierten Endothelzellen als Vektoren

Endothelzellen, isoliert, transduziert und vermehrt, wie in Abschnitt 12.4.) beschrieben, werden in 60 mm Kulturschalen eingesät und mit einem Komplex aus einem weiteren erfindungsgemäßen Plasmid und Superfect (Fa. Quiagen) für 10 min bei 37 °C inkubiert.

Die Herstellung dieses Komplexes erfolgt nach Angabe des Herstellers von Superfect.

Das erfindungsgemäße Plasmid enthalt in Leserichtung von 5' nach 3' folgende DNA-Sequenzen:

### Aktivatorsubeinheit A)

- der Promotor des cdc25C Genes (Nukleinsäuren 290 bis +121; Zwicker et al., EMBO J. 14, 4514 (1995); Zwicker et al., Nucl. Acids Res. 23, 3822 (1995))
- das nukleare Lokalisationssignal (NLS) von SV40 (SV40 Large T, Aminosäuren 126-132; PKKKRKV (SEQ ID NO.: 3), Dingwall et al., TIBS 16, 478 (1991))
- die saure Transaktivierungsdomäne (TAD) von HSV-1 VP16 (Aminosäuren 406 bis 488; Triezenberg et al., Genes Developm. 2, 718 (1988); Triezenberg, Curr. Opin. Gen. Developm. 5, 190 (1995))
- die cDNA für den zytoplasmatischen Teil des CD4 Glycoproteins (Aminosauren 397-435; Simpson et al., Oncogene 4, 1141 (1989); Maddon et al., Cell 42, 93 (1985))

### Aktivatorsubeinheit B

- der Promotor des humanen Endoglingenes (Nukleinsäuren 1 bis 2415; Patentanmeldung D19704301.1)
- das nukleare Lokalisationssignal (NLS) von SV40 (SV40 Large T; Aminosäuren 126-132 PKKKRKV (SEQ ID NO.: 3); Dingwall et al., TIBS 16, 478 (1991))
- die cDNA für die DNA-Bindedomäne des Gal4 Proteins (Aminosäuren 1 bis 147, Chasman und Kornberg, Mol. Cell. Biol. 10, 2916 (1990))
- die cDNA für die CD4 Bindesequenz des p56 Ick Proteins (Aminosäuren 1-71; Shaw et al., Cell 59, 627 (1989); Turner et al., Cell 60, 755 (1990); Perlmutter et al., J. Cell. Biochem. 38, 117 (1988))

### Aktivator-responsive Promotoren

- 10x die Bindesequenz für Gal4-Bindeprotein mit der Nukleotidsequenz 5'-CGGACAATGTTGACCG-3' (SEQ ID NO.: 4, Chasman und Kornberg, Mol. Cell. Biol. 10, 2916 (1989))
- der basale Promotor von SV40 (Nukleinsäuren 48 bis 5191; Tooze (ed). DNA Tumor Viruses (Cold Spring Harbor New York, New York, Cold Spring Harbor Laboratory)

### Effektorgen

- die cDNA für die humane β-Glucuronidase (Nukleotidsequenz 93 bis 1982; Oshima et al., PNAS USA 84, 65 (1987))

Die Funktionsweise der beschriebenen Aktivatorsequenz ist wie folgt:
- Der Promotor cdc25B reguliert zellzyklusspezifisch die Transkription der kombinierten cDNAs für die Aktivierungsdomane von VP16 und dem zytoplasmatischen Teil von CD4 (Aktivierungsuntereinheit A).
- Der Promotor des humanen Endoglingenes reguliert endothelzellspezifisch die Transkription der kombinierten cDNAs für das DNA Bindeprotein von Gal4 und des CD4 bindenden Teiles des p56 Ick-Proteins (Aktivierungsuntereinheit B).
- Die Expressionsprodukte der Aktivatorsubeinheiten A und B dimerisieren durch Bindung der CD4-Domäne an die p56 Ick-Domäne.
- Das dimere Protein stellt einen chimären Transkriptionsfaktor für den Aktivator-responsiven Promotor (DNA-Sequenz für die Gal4-Bindedomänen/den SV40 Promotor) für die Transkription des Effektorgenes (= Luciferasegen) dar.

Die Verknüpfung der einzelnen Bestandteile des Konstruktes erfolgt über geeignete Restriktionsstellen, die über PCR-Amplifikation an den Termini der verschiedenen Elemente mitgeführt werden. Die Verknüpfung erfolgt mit Hilfe von dem Fachmann bekannten, für die Restriktionsstellen spezifischen Enzyme und DNA-Ligasen. Diese Enzyme sind käuflich zu erwerben.

Mit Hilfe dieser Enzyme wird das so hergestellte Nukleotidkonstrukt in den pXP2 Plasmidvektor (Nordeen, BioTechniques 6, 454 (1988)) einkloniert. Nach Inkubation der mononukleären Blutzellen mit dem Superfect/Plasmid Komplex werden die Blutzellen gewaschen und in Zellkulturmedium kultiviert wie im Abschnitt 12.2.) beschrieben.

Nach 6 Tagen wird die Menge an β-Glucuronidase , produziert von den Endothelzellen, mit Hilfe von 4-Methylumbelliferyl-β-glucuronid als Substrat gemessen.

Zur Überprüfung der Zellzyklusspezifität werden Endothelzellen durch Entzug von Methionin über 48 Stunden in G₀/G₁ synchronisiert. Der DNA-Gehalt der Zellen wird nach Anfärbung mit Hoechst 33258 im Fluoreszenzaktivierungs-Cell Sorter bestimmt (Lucibello et al., EMBO J. 14, 132 (1995)).

Folgende Ergebnisse werden erzielt:
In transfizierten Endothelzellen kann keine Zunahme der β-Glucuronidase im Vergleich zu nichttransfizierten Fibroblasten ermittelt werden.
Transfizierte Endothelzellen exprimieren deutlich mehr β-Glucuronidase als nichttransfizierte Endothelzellen.
Proliferierende Endothelzellen (DNA > 2S; S = einfacher Chromosomensatz) sekretieren deutlich mehr β-Glucuronidase als in G₀/G₁ synchronisierte Endothelzellen (DNA = 2S).
   Somit führt die beschriebene Aktivator-responsive Promotoreinheit zu einer zellspezifischen, zellzyklusabhängigen Expression des Strukturgenes β-Glucuronidase.

Endothelzellen gemäß der vorliegenden Erfindung ermöglichen nach lokaler Applikation beispielsweise an den Ort des Tumors oder nach intrakranieller bzw. Subarachnoidaler Gabe oder systemischer, bevorzugt intravenöser oder intraarterieller Gabe, daß diese Endothelzellen sich bevorzugt an Regionen mit Gefäßschaden ansiedeln und durch die Zellzyklus- und Endothelzellspezifität der Aktivator-responsiven Promotoreinheit vorwiegend, wenn nicht ausschließlich, nur proliferierende Endothelzellen β-Glucuronidase ausscheiden. Diese β-Glucuronidase spaltet ein nunmehr injiziertes, gut verträgliches Doxorubicin. Dieses hemmt die Endothelzellproliferation und wirkt zytostatisch auf diese Zellen wie auch auf benachbarte Tumorzellen. Hierdurch kommt es zur Hemmung des Tumorwachstums.

## Patentansprüche

1. Zellen zur Anwendung in der Gentherapie erhältlich durch
a) Isolierung von mononukleären nichtadhärenten Zellen aus dem Blut oder zellhaltigen Flüssigkeiten des Körpers;
b) Kultivierung der in Schritt a) gewonnenen Zellen in einem Zellkulturmedium enthaltend Ganglioside, Phospholipide, Glykolipide und/oder Wachstumsfaktoren Wachstumsfaktoren für Endothelzellen, einschließlich solcher Faktoren, welche Differenzierung, Überleben, Migration und/oder Vaskularisation beeinflussen;
c) wahlweise Immortalisierung der in Schritt a) oder b) gewonnenen Zellen durch Transformation mit einem Onkogen, Aktivierung eines Onkogens oder Inaktivierung eines Suppressorgens; und
d) wahlweise Transfektion der in Schritt a) und b) oder in Schritt c) gewonnenen Zellen mit einem Nukleinsäurekonstrukt für die Gentherapie, enthaltend ein Effektorgen, welches durch geeignete Promotorsysteme zielzellspezifisch, zellzyklusspezifisch, virusspezifisch und/oder durch Hypoxie aktiviert werden kann.

2. Zellen gemäß Anspruch 1, wobei die Zellen CD34-, CD14-, CD11-, CD11b-, CD13-, CD64- oder CD68-positiv oder Endothelzellen sind.

3. Zellen gemäß einem der Ansprüche 1 oder 2, dadurch gekennzeichnet, daß diese Zellen aus dem Blut in Venen, Kapillaren, Arterien, Nabelschnur oder Plazenta, aus dem Knochenmark, der Milz, den Lymphknoten, dem Peritonealraum, dem Pleuralraum, der Lymphe, den Venen, Arterien, Kapillaren und/oder der Bindegewebsflüssigkeit stammen.

4. Zellen gemäß Anspruch 3, dadurch gekennzeichnet, daß der Wachstumsfaktor in Schritt b) von Anspruch 1 ausgewählt wird aus der Gruppe enthaltend ECGF, FGFα, FGFβ, VEGF, ECAF, IGF-1; IGF-2; SC-3; EGF; SCF, TGFβ, Angiogenin, Pleiotrophin und Flt-3-Ligand.

5. Zellen gemäß einem der Ansprüche 3 oder 4, dadurch gekennzeichnet, daß das Onkogen in Schritt c) von Anspruch 1 derartig mutiert ist, daß das Onkogen-Genprodukt noch voll den Zellzyklus aktivieren kann, aber diese Aktivierung des Zellzyklus nicht mehr durch zelluläre Inhibitoren inhibierbar ist.

6. Zellen gemäß Anspruch 5, dadurch gekennzeichnet, daß das Onkogen ausgewählt wird aus der Gruppe enthaltend mutiertes cdk-4, cdk-6 und cdk-2.

7. Zellen gemäß Anspruch 6, dadurch gekennzeichnet, daß die Nukleotidsequenz für cdk-4 in der Position 24 derartig mutiert ist, daß das eigentlich kodierte Arginin ausgetauscht ist gegen ein Cystein.

8. Zellen gemäß einem der Ansprüche 3 oder 4, dadurch gekennzeichnet, daß die Inaktivierung eines Suppressorgenes gemäß Schritt c) in Anspruch 1 durch Transformation der Zellen mit einer Nukleinsäuresequenz kodierend für ein Protein, welches mindestens ein Suppressorgen-Genprodukt inaktiviert, erreicht wird.

9. Zellen gemäß Anspruch 8, dadurch gekennzeichnet, daß das Suppressorgen-Genprodukt inaktivierende Protein ausgewählt wird aus der Gruppe enthaltend das E1A-Protein des Adenovirus, das E1B-Protein des Adenovirus, das large T-Antigen des SV40-Virus, das E6-Protein des Papillomavirus, das E7-Protein des Papillomavirus, das MDM-2-Protein und ein Protein enthaltend mindestens eine Aminosäuresequenz LXDXLXXL-II-LXCXEXXXXXSDDE, bei welcher X eine variable Aminosäure und -II- eine beliebige Aminosäurekette von 7-80 Aminosäuren bedeutet.

10. Zelle gemäß einem der Ansprüche 5 bis 9, dadurch gekennzeichnet, daß die Zelle eine Endothelzelle ist und das zur Transformation benutzte Onkogen oder die zur Inaktivierung des Suppressorgens benutzte Nukleinsäuresequenz verknüpft ist mit einer endothelspezifischen Aktivierungssequenz, welche die Transkription des Onkogens oder der genannten Nukleotidsequenz steuert.

11. Zellen gemäß einem der Ansprüche 1 bis 10, dadurch gekennzeichnet, daß das Nukleinsäurekonstrukt in Schritt d) im Anspruch 1
• mindestens eine uneingeschränkt aktivierbare, eine endothelzellspezifische, virusspezifische, eine metabolisch aktivierbare und/oder eine zellzyklusspezifisch aktivierbare Aktivierungssequenz und
• mindestens ein Effektorgen, dessen Expression von der Aktivierungssequenz gesteuert wird,
enthält.

12. Zellen nach Anspruch 11, dadurch charakterisiert, daß die Expression des Effektorgenes durch mindestens zwei gleiche oder verschiedene Aktivierungssequenzen gesteuert wird.

13. Zellen nach einem der Ansprüche 11 oder 12, dadurch gekennzeichnet, daß die Aktivierung der Aktivierungssequenz selbstverstärkend und/oder pharmakologisch kontrollierbar ist.

14. Zellen nach einem der Ansprüche 12 oder 13, dadurch charakterisiert, daß die zweite Aktivatorsequenz ausgewählt ist aus der Gruppe enthaltend Promotorsequenzen von Viren wie HBV, HCV, HSV, HPV, EBV, HTLV, CMV oder HIV; Promotor- oder Enhancersequenzen aktiviert durch Hypoxie oder zellzyklusspezifische Aktivierungssequenzen der Gene für cdc25C, cdc25B, Cyclin A, cdc2, E2F-1, B-myb und DHFR; Bindesequenzen für zellproliferationsabhängig auftretende oder aktivierte Transkriptionsfaktoren wie Monomere oder Multimere der Myc E-Box.

15. Zellen nach einem der Ansprüche 13 oder 14, dadurch gekennzeichnet, daß es sich bei dem Effektorgen um ein Gen handelt, das für einen Wirkstoff kodiert, der ausgewählt ist aus der Gruppe enthaltend Cytokine, Chemokine, Wachstumsfaktoren, Rezeptoren für Cytokine, Chemokine oder Wachstumsfaktoren, antiproliferativ oder zytostatisch oder apoptotisch wirkende Proteine, Antikörper, Antikörperfragmente, Angiogeneseinhibitoren, Peptidhormone, Gerinnungsfaktoren, Gerinnungshemmer, fibrinolytische Proteine, auf den Blutkreislauf wirksame Peptide oder Proteine, Blutplasmaproteine und Antigene von Infektionserregern oder von Zellen oder von Tumoren, wobei das ausgewählte Antigen eine Immunreaktion bewirkt.

16. Zellen nach einem der Ansprüche 13 oder 14, dadurch gekennzeichnet, daß es sich bei dem Effektorgen um ein Gen handelt, das für ein Enzym kodiert, welches eine Vorstufe eines Pharmakons in ein Pharmakon spaltet.

17. Zellen nach einem der Ansprüche 13 oder 14, dadurch gekennzeichnet, daß es sich bei dem Effektorgen um ein Gen handelt, das für ein Ligand-Wirkstoff-Fusionsprotein oder ein Ligand-Enzym-Fusionsprotein kodiert, wobei der Ligand ausgewählt ist aus einer Gruppe umfassend Cytokine, Wachstumsfaktoren, Antikörper, Antikörperfragmente, Peptidhormone, Mediatoren, Zelladhäsionsproteine und LDL-Rezeptor bindende Proteine.

18. Zellen nach einem der vorhergehenden Ansprüche, dadurch gekennzeichnet, daß es sich bei dem in die Endothelzelle eingeführten Nukleinsäurekonstrukt um DNS handelt.

19. Zellen gemäß Anspruch 18, dadurch gekennzeichnet, daß das Nukleinsäurekonstrukt eingefügt ist in einen Vektor.

20. Zellen gemäß Anspruch 19, dadurch gekennzeichnet, daß es sich um einen Plasmidvektor handelt.

21. Zellen gemäß Anspruch 19, dadurch gekennzeichnet, daß es sich um einen viralen Vektor handelt.

22. Zellen nach einem der Ansprüche 1 bis 21, dadurch gekennzeichnet, daß sie äußerlich, peroral, intravesikal, nasal, intrabronchial oder in den Magen-Darm-Trakt verabreicht oder in ein Organ, in eine Körperhöhle, in die Muskulatur, subkutan oder in den Blutkreislauf injiziert werden zur Prophylaxe oder Therapie einer Erkrankung.

23. Verwendung einer Zelle nach einem der Ansprüche 1 bis 22 zur Herstellung eines Heilmittels zur Behandlung einer Erkrankung ausgewählt aus der Gruppe enthaltend Tumore, Leukämien, Autoimmunerkrankungen, Allergien, Arthritiden, Entzündungen, Organabstoßungen, Transplantat gegen Wirt-Reaktionen, Blutgerinnungserkrankungen, Kreislauferkrankungen, Blutarmut, Infektionen, Hormonerkrankungen und ZNS-Schäden.

24. Verfahren zur Herstellung einer Zelle gemäß einem der Ansprüche 1 bis 22, dadurch gekennzeichnet, daß folgende Schritte durchgeführt werden:
a) Isolierung von Zellen aus dem Blut oder zellhaltigen Flüssigkeiten des Körpers;
b) Kultivierung der in Schritt a) gewonnenen Zellen in einem Zellkulturmedium enthaltend Ganglioside, Phospholipide, Glykolipide und/oder Wachstumsfaktoren;
c) Wahlweise Immortalisierung der in Schritt a) oder b) gewonnenen Zellen durch Transformation mit einem Onkogen, Aktivierung eines Onkogens oder Inaktivierung eines Suppressorgens;
d) Transfektion der in Schritt a) und b) oder in Schritt c) gewonnenen Zellen mit einem Nukleinsäurekonstrukt für die Gentherapie, enthaltend ein Effektorgen, welches durch geeignete Promotorsysteme zielzellspezifisch, zellzyklusspezifisch, virusspezifisch und/oder durch Hypoxie aktiviert werden kann.

25. Arzneimittel, enthaltend Zellen gemäß einem der Ansprüche 1 bis 22.

26. Zellen erhältlich gemäß Anspruch 1 zur Endothelisierung verletzter Gefäße.


SEQUENZPROTOKOLL

(1) ALLGEMEINE INFORMATION:

(i) ANMELDER:
(A) NAME: Hoechst Marion Roussel Deutschland GmbH
(B) STRASSE: -
(C) ORT: Frankfurt
(D) BUNDESLAND: -
(E) LAND: Deutschland
(F) POSTLEITZAHL: 65926
(G) TELEPHON: 069-305-3005
(H) TELEFAX: 069-35-7175
(I) TELEX: -

(ii) ANMELDETITEL: Genetisch veraenderte Zellen und deren
Verwendung in der Prophylaxe oder Therapie von
Erkrankungen

(iii) ANZAHL DER SEQUENZEN: 4

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 23 Aminosäuren
(B) ART: Aminosäure
(C) STRANGFORM: Einzel
(D) TOPOLOGIE: linear

(ii) ART DES MOLEKÜLS: Peptid

(ix) MERKMALE:
(A) NAME/SCHLÜSSEL: Peptide
(B) LAGE: 1..23
(D) SONSTIGE ANGABEN: /note= "Xaa = eine der 20 genetisch
kodierbaren Aminosaeuren;
Xaa* = Aminosaeurekette bestehend aus 7-80 Aminosäuren Xaa.

(xi) SEQUENZBESCHREIBUNG: SEQ ID NO: 1:

Leu Xaa Asp Xaa Leu Xaa Xaa Leu Xaa* Leu Xaa Cys Xaa Glu Xaa Xaa
1               5                    10                  15

Xaa Xaa Xaa Ser Asp Asp Glu
            20

(2) INFORMATION ZU SEQ ID NO: 2:

(i) SEQUENZ CHARAKTERISTIKA:
(A) LÄNGE: 26 Basenpaare
(B) ART: Nukleinsäure
(C) STRANGFORM: Doppel
(D) TOPOLOGIE: linear

(ii) ART DES MOLEKÜLS: DNS

(ix) MERKMALE:
(A) NAME/SCHLÜSSEL: Myc E-Box
(B) LAGE: 1..26

(xi) SEQUENZBESCHREIBUNG: SEQ ID NO: 2:

GGAAGCAGAC CACGTGGTCT GCTTCC 26

(2) INFORMATION ZU SEQ ID NO: 3:

(i) SEQUENZ CHARAKTERISTIKA:
(A) LÄNGE: 7 Aminosäuren
(B) ART: Aminosäure
(C) STRANGFORM: Einzel
(D) TOPOLOGIE: linear

(ii) ART DES MOLEKÜLS: Peptid

(ix) MERKMALE:
(A) NAME/SCHLÜSSEL: NLS von SV 40
(B) LAGE: 1..7

(xi) SEQUENZBESCHREIBUNG: SEQ ID NO: 3:

Pro Lys Lys Lys Arg Lys Val
1               5

(2) INFORMATION ZU SEQ ID NO: 4:

(i) SEQUENZ CHARAKTERISTIKA:
(A) LÄNGE: 16 Basenpaare
(B) ART: Nukleinsäure
(C) STRANGFORM: Einzel
(D) TOPOLOGIE: linear

(ii) ART DES MOLEKÜLS: DNS

(ix) MERKMALE:
(A) NAME/SCHLÜSSEL: Bindesequenz für Gal 4
(B) LAGE: 1..16

(xi) SEQUENZBESCHREIBUNG: SEQ ID NO: 4:

CGGACAATGT TGACCG 16